# EUROPÄISCHE PATENTSCHRIFT

(11) **EP 3 402 573 B1**
(45) Veröffentlichungstag und Bekanntmachung des Hinweises auf die Patenterteilung: **01.03.2023**
(21) Anmeldenummer: 17700936.2
(22) Anmeldetag: 13.01.2017
(51) Int. Cl.: A61Q 5/00, A61Q 5/12, A61Q 19/00, A61Q 19/10, A61K 8/896, C11D 17/00, A23C 9/12, A23J 3/34, C11D 3/38, C11D 9/40

(54) **AMINOSÄUREN-HALTIGE ZUSAMMENSETZUNG**
COMPOSITION CONTAINING AMINO ACIDS
COMPOSITION CONTENANT DES ACIDES AMINÉS

(30) Priorität: 13.01.2016 DE 102016000286; 13.01.2016 DE 202016000228 U
(43) Veröffentlichungstag der Anmeldung: 21.11.2018
(73) Patentinhaber: Priemer, Wolfgang, 32257 Bünde (DE); Finke, Robert, 32312 Lübbecke (DE)
(72) Erfinder: Priemer, Wolfgang, 32257 Bünde (DE); Finke, Robert, 32312 Lübbecke (DE)
(74) Vertreter: Wallinger, Michael
(86) Internationale Anmeldenummer: PCT/EP2017/050683
(87) Internationale Veröffentlichungsnummer: WO 2017/121859

(56) Entgegenhaltungen:
- EP-A2- 0 457 565
- WO-A1-2014/130007
- CN-A- 104 664 375
- DE-A1-102015 002 418
- GB-A- 1 308 690
- JP-A- H02 234 642
- JP-A- H06 165 655
- US-A- 4 358 465

## Beschreibung

Die vorliegende Erfindung betrifft eine Zusammensetzung aufweisend freie Aminosäuren, Oligopeptide und Polypeptide sowie deren Herstellung durch enzymatischen Verdau natürlicher Eiweißquellen. Die Erfindung wird insbesondere im Zusammenhang mit der Verwendung in der Lebensmittel/Getränkeherstellung, der Kosmetik- und Seifenherstellung, bzw. der Herstellung von Körperpflegeprodukten und Schlankheitsmitteln beschrieben. Es wird darauf hingewiesen, dass die Erfindung auch in anderen Bereichen Anwendung finden kann, wie zum Beispiel in der Herstellung von Tiernahrung.

Die 20 proteinogenen Aminosäuren sind die Bausteine aller zellulären Proteine umfassend Strukturproteine, Transport-Proteine, Speicher-Proteine, Immunoglobuline und Enzyme. Die meisten höheren Organismen sind auf die Zufuhr einzelner Aminosäuren mit der Nahrung angewiesen (essentielle Aminosäuren). Beim Menschen sind dies Isoleucin, Leucin, Lysin, Methionin, Phenylalanin, Threonin, Tryptophan und Valin. Eine ausreichende Versorgung mit Aminosäuren ist für die Proteinsynthese essentiell. Die Proteinsynthese ist dabei quantitativ durch die jeweils limitierende Aminosäure begrenzt. Proteinsynthese ist für alle zellulären Prozesse unerlässlich. Eine mangelhafte Versorgung mit Aminosäuren führt zu Mangelkrankheiten, Marasmus und einer deutlichen Schwächung des Immunsystems und hat Auswirkungen auf die Vitalkraft und das äußere Erscheinungsbild von Haaren, Nägeln und Haut. Eine ausreichende Versorgung mit Aminosäuren ist daher insbesondere für Menschen wichtig, die Sport treiben, sowie für Menschen, die ihr Gewicht reduzieren möchten oder aufgrund bestehender gesundheitlicher Probleme eine mangelnde Nahrungsaufnahme / Nahrungsverwertung zeigen und dennoch eine schnelle und vollständige Versorgung mit Aminosäuren und Peptiden / Proteinen erreichen möchten, um die Abwehrkraft und Muskulatur zu erhalten oder die Muskelbildung zu steigern.

Aminosäuren sind außerdem Bestandteil des NMF (Natural Moisterizing Factor) - eine Hautkomponente, die für die Feuchtigkeitsregulierung und anderer Funktionen der Haut verantwortlich ist, sowie des Collagens, eines der wichtigsten Strukturproteine des Bindegewebes und der Haut. Des Weiteren stabilisieren freie Aminosäuren den Säureschutzmantel der Haut. Aus diesen Gründen werden Aminosäuren, Peptide und Proteine in der Haut- und Haarkosmetik eingesetzt.

Aminosäuren werden über die Nahrung vor allem in Form von Proteinen aufgenommen, die anschließend in Magen und Darm hydrolysiert werden. Die hierbei entstehenden Aminosäuren, wie auch Di- und Tripeptide werden im Darm resorbiert. Es ist bekannt, dass durch die Einnahme bereits vorverdauter also hydrolysierter Proteine die Bioverfügbarkeit deutlich beschleunigt werden kann. Freie Aminosäuren können praktisch unmittelbar nachdem sie im Darm ankommen resorbiert werden. Insbesondere Sportmediziner verabreichen Aminosäuren nach dem Training und verweisen auf eine Zeitspanne von ca. 30 Minuten für die Aufnahme der die Aminosäuren in den Blutkreislauf. Im Vergleich hierzu würde der Verzehr von Eiern zu einer Aminosäureaufnahme nach mehr als fünf Stunden führen. In der Zwischenzeit können Versorgungslücken auftreten und das Immunsystem kann geschwächt werden.

Die DE 196 06 439 A beschreibt ein Verfahren zum enzymatischen Abbau von Proteinen. In dem beschriebenen Verfahren werden vorzugsweise nicht aufgereinigte Proteasen, welche gesondert erzeugt und bereitgestellt werden, sondern Protease-produzierende Mikroorganismen eingesetzt.

Die WO 2014/130007 A1 beschreibt proteolytische Zusammensetzungen für raschen und weitgehenden Verdau von Proteinzusätzen. Die proteolytischen Verbindungen enthalten Subtilisin Carlsberg und optional Bromelin und der proteolytische Verdau findet erst im Körper der Probanden statt.

Der Erfindung liegt die Aufgabe zugrunde, ein alternatives Verfahren zur Herstellung einer Zusammensetzung aufweisend freie Aminosäuren, Oligopeptide und Polypeptide zur Verfügung zu stellen.

Das wird erfindungsgemäß durch die Lehre des unabhängigen Anspruchs 1 erreicht. Zu bevorzugende Weiterbildungen der Erfindung sind Gegenstand der Unteransprüche.

Gemäß einem ersten Aspekt betrifft die Erfindung ein Verfahren zur Herstellung einer Zusammensetzung aufweisend freie Aminosäuren, Oligopeptide und Polypeptide.

Ein erfindungsgemäßes Verfahren weist zumindest den Schritt der Zugabe von mindestens zwei verschiedenen Peptidasen, welche in aufgereinigter Form vorliegen, zu einer Zusammensetzung, welche mindestens ein Protein aufweist, auf, wobei mindestens eine Exopeptidase und mindestens eine Endopeptidase zu der Zusammensetzung aufweisend mindestens ein Protein zugegeben werden, und wobei
(a) die mindestens zwei verschiedenen Peptidasen mikrobiellen Ursprungs sind;
(b) die Zusammensetzung aufweisend mindestens ein Protein eine Proteinkonzentration von 5 bis 30 Gewichtsprozent bezogen auf das Gesamtgewicht der Zusammensetzung aufweisend mindestens ein Protein aufweist;
(c) die Zusammensetzung aufweisend mindestens ein Protein bei Zugabe der mindestens zwei verschiedenen Peptidasen einen pH-Wert von 4 bis 6 hat und bei einer Temperatur von 40°C bis 60°C mit den mindestens zwei verschiedenen Peptidasen inkubiert wird; und
(d) die Gesamtmenge an zugegebener Peptidase jeweils von 2 bis 10 Gramm pro 100 Gramm des mindestens einen Proteins in der Zusammensetzung aufweisend mindestens ein Protein beträgt.

Im Rahmen der vorliegenden Erfindung wurde gefunden, dass die Ausbeute an freien Aminosäuren und/oder an Oligopeptiden mit einer Länge von bis zu 10 Aminosäuren bei dem enzymatischen Verdau mindestens eines Proteins bei entsprechender Gestaltung verbessert werden kann, wenn dem mindestens einen Protein mindestens zwei verschiedene Peptidasen in aufgereinigter Form zugesetzt werden, anstatt Peptidase-produzierende Bakterien zuzusetzen. insbesondere wurde gefunden, dass durch die Verwendung zweier verschiedener aufgereinigter Peptidasen bei entsprechender Gestaltung des Herstellungsprozesses Zusammensetzungen mit allen acht für den Menschen essentiellen Aminosäuren oder sogar mit allen zwanzig proteinogenen Aminosäuren erhalten werden können. Durch das erfindungsgemäße Herstellungsverfahren erzeugte Zusammensetzungen eignen sich daher, Versorgungslücken an einzelnen Aminosäuren zu schließen und können so Mangelerkrankungen oder Mangelerscheinungen wie zum Beispiel der Haut oder des Haares, Marasmus und einer Schwächung des Immunsystems vorbeugen.

Gegebenenfalls kann die Zusammensetzung außerdem variierende Mengen des ungespaltenen Ausgangsproteins, abhängig von der Fermentationsdauer und -effizienz sowie der eingesetzten Menge an Peptidasen, aufweisen.

Des Weiteren wurde gefunden, dass die Verfahrensdauer des erfindungsgemäßen Verfahrens bei entsprechender Gestaltung gegenüber der bakteriellen Fermentation von Proteinen, also dem enzymatischen Verdau von Proteinen durch die Zugabe von Peptidase-produzierenden Bakterien, verkürzt werden kann. Dadurch lassen sich die Produktionszyklen effizienter gestalten und der Einsatz großer Fermentationstanks wird entbehrlich.

Aminosäure-haltige Zusammensetzungen, welche durch konventionelle bakterielle Fermentation, also durch die Zugabe Peptidase-produzierender Bakterien erhalten werden, haben typischerweise einen ausgeprägten Bittergeschmack. Zudem wird häufig ein starker Fäulnisgeschmack beobachtet, der solche Zusammensetzungen für den menschlichen Verzehr praktisch ungeeignet machen kann. Sowohl Fäulnis- als auch Bittergeschmack können durch das erfindungsgemäße Verfahren bei entsprechender Gestaltung überraschender Weise verringert oder sogar vollständig verhindert werden. Die durch das erfindungsgemäße Verfahren hergestellten Aminosäure-haltigen Zusammensetzungen können bei entsprechender Gestaltung des Verfahrens frei von Fremdgeschmack und frei von käsigem Bei- oder Nachgeschmack sein.

Die Begriffe "Eiweiß" und "Protein" werden vorliegend synonym verwendet. Unter diesen Begriffen sind im Sinne der Erfindung organische Moleküle, welche aus Aminosäuren aufgebaut sind, die durch Peptidbindungen miteinander verbunden sind, zu verstehen. Proteine im Sinne der Erfindung sind unverdaut, also nicht durch Hydrolyse eines größeren Proteins entstanden. Die Größe von Proteinen im Sinne der Erfindung ist nicht beschränkt, vorzugsweise weisen Proteine aber mindestens 100 Aminosäuren auf. Proteine im Sinne der Erfindung umfassen insbesondere tierische, pflanzliche und fungale Proteine, die Verwendung von Proteinen bakteriellen oder sonstigen mikrobiellen Ursprungs als Ausgangssubstrat für die enzymatische Hydrolyse ist aber nicht ausgeschlossen. Proteine im Sinne der Erfindung sind hinsichtlich ihrer Sekundär-, Tertiär- und Quartärstruktur in keiner Weise beschränkt und umfassen globuläre und fibrilläre Proteine. Auch hinsichtlich ihrer Funktion sind Proteine im Sinne der Erfindung nicht beschränkt und umfassen unter anderem Strukturproteine, Transport-Proteine, Speicher-Proteine, Immunoglobuline und Enzyme.

Unter dem Begriff "Peptidase" im Sinne der Erfindung sind Enzyme, welche Proteine oder Peptide spalten können, zu verstehen. Peptidasen katalysieren die Hydrolyse von Peptidbindungen in Peptiden und Proteinen. Die Begriffe "Protein- oder Peptid-Spaltung", "Protein- oder Peptid-Verdau" und "Hydrolyse von Peptidbindungen" werden vorliegend synonym verwendet. Unter dem Begriff 'Fermentation von Proteinen, Peptiden oder Eiweißquellen" im Sinne der Erfindung ist die enzymatische Spaltung von Proteinen und Peptiden zu verstehen. Unter dem Begriff "bakterielle Fermentation" ist die Fermentation durch Zugabe von Peptidase produzierenden Bakterien zu verstehen. Unter dem Begriff "enzymatische Fermentation" ist die Fermentation durch Zugabe von aufgereinigten Peptidasen zu verstehen. Der Begriff "Peptidase" wird vorliegend synonym mit den Begriffen "Protease" und "Proteinase" verwendet. Im Sinne der Erfindung ist unter dem Begriff "Peptidase" das Enzym in aufgereinigter Form zu verstehen, wenn nicht ausdrücklich etwas anderes erwähnt ist. Unter dem Begriff "in aufgereinigter Form" ist das Vorliegen des Enzyms in freier Form in einer Enzympräparation zu verstehen. Die Herstellung solcher Enzympräparationen kann auf jegliche Art erfolgen, zum Beispiel durch Isolierung und Aufreinigung des Enzyms nach homologer oder heterologer Expression in Mikroorganismen. Die Verwendung aufgereinigter Peptidasen bietet gegenüber der Verwendung von Peptidase-produzierenden Bakterien den Vorteil besserer Kontrollierbarkeit und Reproduzierbarkeit des Prozesses. Darüber hinaus kann das Risiko einer Kontamination mit Bakteriophagen vermieden werden.

Unter dem Begriff "freie Aminosäuren" im Sinne der vorliegenden Erfindung sind Aminosäuren, welche keine Peptidbindungen zu anderen Aminosäuren aufweisen, zu verstehen. Freie Aminosäuren im Sinne der Erfindung entstehen durch den enzymatischen Verdau von Proteinen oder Peptiden, einschließlich Oligopeptiden, durch die enzymatische Hydrolyse von Peptidbindungen in diesen Proteinen oder Peptiden.

Unter dem Begriff "Peptid" im Sinne der Erfindung ist ein Spaltprodukt eines Proteins zu verstehen, welches durch enzymatische Hydrolyse einer oder zwei der im Protein vorhanden Peptidbindungen entstanden ist.

Unter dem Begriff "Oligopeptid" im Sinne der Erfindung ist ein Peptid, also ein durch enzymatischen Verdau hergestelltes Spaltprodukt eines Proteins, zu verstehen, welches aus 2 bis 10 Aminosäuren besteht. Der Begriff Oligopeptid umfasst somit die Begriffe Dipeptid (Peptid bestehend aus zwei Aminosäuren), Tripeptid (Peptid bestehend aus drei Aminosäuren), Tetrapeptid (Peptid bestehend aus vier Aminosäuren) und Pentapeptid (Peptid bestehend aus fünf Aminosäuren).

Unter dem Begriff "Polypeptid" im Sinne der Erfindung ist ein Peptid, also ein durch enzymatischen Verdau hergestelltes Spaltprodukt eines Proteins, zu verstehen, welches aus mindestens 11 Aminosäuren besteht. Nach oben hin ist die Länge des Polypeptids im Sinne der Erfindung lediglich dadurch begrenzt, dass es zumindest eine Aminosäure weniger aufweist, als das Ausgangsprotein, durch dessen Aufspaltung das Polypeptid entstanden ist.

Zusammensetzungen, welche mittels des erfindungsgemäßen Herstellungsverfahrens erzeugt werden, haben gegenüber herkömmlichem Nahrungseiweiß den Vorteil, dass Aminosäuren und Oligopeptide deutlich schneller im Körper resorbiert werden können als ungespaltene Proteine. Freie Aminosäuren werden praktisch sofort nachdem sie den Darm erreichen resorbiert. Je nach Menge der aufgenommenen Aminosäuren dauert die Resorption beispielsweise bis zu einer Stunde an. Oligopeptide werden vor ihrer Resorption noch in freie Aminosäuren gespalten, wodurch sich eine zeitliche Verzögerung der Resorption von etwa einer halben Stunde ergibt. Bei Polypeptiden dauert die Aufspaltung im Darm bis zu freien Aminosäuren in etwa ein bis zwei Stunden, je nach Länge der Polypeptide. Das erfindungsgemäße Herstellungsverfahren liefert somit Zusammensetzungen, welche eine schnelle, nahezu sofortige, stufenweise Versorgung mit Aminosäuren bewirkt. Durch das gleichzeitige Vorliegen freier Aminosäuren, kurzer Oligopeptide, längerer Polypeptide und gegebenenfalls noch ungespaltener Proteine in der Zusammensetzung wird eine praktisch sofort einsetzende und über mehrere Stunden anhaltende, lückenlose Versorgung mit Aminosäuren ermöglicht.

In Körperpflegeprodukten können die durch das erfindungsgemäße Verfahren erzeugten Aminosäuren und Oligopeptide als Eiweißquelle für den Aufbau von Collagen und Elastin dienen, welche natürlicherweise in der Haut vorkommen. Collagene besitzen ein hohes Molekulargewicht und können daher die Haut nicht penetrieren. Sie wirken hautstraffend und binden in hohem Maße Wasser. Lösliches Collagen beschleunigt die Heilung von Wunden und die spontane Re-Epithelisierung der Haut. Hierbei stimuliert es die Wiederherstellung von Gewebe und die Entstehung von Makromolekülen.

Peptide sind außerdem Teil des Bindegewebes, der Haare und der Nägel. Sie wirken als Botenstoffe, Hormone oder Co-Enzyme und sind wesentliche Akteure in nahezu allen biologischen Prozessen. So beschleunigen sie Heilprozesse und die Zellerneuerung. Peptide haben häufig regulierende Funktionen. Insulin zum Beispiel ist ein Peptid, welches den Zuckerstoffwechsel im Körper steuert.

Auf der Hautoberfläche werden Peptide, insbesondere Oligopeptide, teilweise zu freien Aminosäuren abgebaut und unterstützen somit den natürlichen Feuchtigkeitsgehalt der Haut. Aminosäuren sind als Bestandteil des NMF (Natural Moisterizing Factor) wichtig für die Feuchtigkeitsregulierung und andere Funktionen der Haut. Des Weiteren stabilisieren freie Aminosäuren den Säureschutzmantel der Haut.

In dem erfindungsgemäßen Verfahren werden mindestens eine Exopeptidase und mindestens eine Endopeptidase zu der Zusammensetzung aufweisend mindestens ein Protein zugegeben. Dies bietet den Vorteil, dass der Anteil an freien Aminosäuren und/oder Oligopeptiden auf Kosten der Polypeptide und/oder des mindestens einen Ausgangsproteins, also des mindestens einen Proteins in der Zusammensetzung aufweisend mindestens ein Protein, gegenüber der Verwendung von ausschließlich Endo- oder ausschließlich Exopeptidasen bei gleicher Gesamtmenge an eingesetzten Peptidasen und bei gleicher Verfahrensdauer erhöht werden kann. Dies bietet insbesondere den Vorteil, dass bei entsprechender Ausgestaltung ein optimales Verhältnis von freien Aminosäuren, Oligopeptiden und Polypeptiden erzielt werden kann.

Unter dem Begriff "Endopeptidase" im Sinne der Erfindung ist eine Peptidase zu verstehen, welche die hydrolytische Spaltung von Peptidbindungen im Inneren einer Peptidkette katalysieren kann. Die Spaltprodukte weisen jeweils mindestens zwei Aminosäuren, typischer weise mehr Aminosäuren, auf.

Unter dem Begriff "Exopeptidase" im Sinne der Erfindung ist eine Peptidase zu verstehen, welche die Abspaltung einzelner Aminosäuren an den Enden einer Peptidkette katalysieren kann.

Eine optimale Zusammensetzung zeichnet sich durch einen hohen Anteil an freien Aminosäuren und Oligopeptiden aus. In der durch den Herstellungsprozess erzeugten Zusammensetzung sind 0,5 bis 25 Gewichtsprozent freie Aminosäuren und 25 bis 65 Gewichtsprozent Oligopeptide und vorzugsweise maximal 40 Gewichtsprozent ungespaltenes Ausgangsprotein, bezogen auf das Gesamtgewicht von freien Aminosäuren, Oligopeptiden, Polypeptiden und Proteinen ohne die zugesetzten Peptidasen, enthalten. Besonders bevorzugt sind in der durch den Herstellungsprozess erzeugten Zusammensetzung 2 bis 20 Gewichtsprozent freie Aminosäuren und maximal 10 Gewichtsprozent ungespaltenes Ausgangsprotein, bezogen auf das Gesamtgewicht von freien Aminosäuren, Oligopeptiden, Polypeptiden und Proteinen ohne die zugesetzten Peptidasen, enthalten. Im Vergleich hierzu werden durch die konventionelle Fermentation von Eiweißquellen durch Peptidase-produzierende Bakterien abhängig von der Fermentationsdauer, welche üblicherweise einige Tage bis zu mehreren Wochen beziehungsweise sogar Jahre, wie zum Beispiel bei dem althergebrachten KOSO-Verfahren aus Japan, beträgt, nur etwa 1 bis maximal 12 Gewichtsprozent an freien Aminosäuren bezogen auf das bezogen auf das Gesamtgewicht von freien Aminosäuren, Oligopeptiden, Polypeptiden und Proteinen.

Die Bestimmung des Anteils an freien Aminosäuren und Oligopeptiden in der hergestellten Zusammensetzung erfolgt durch die im Nachfolgenden beschriebene Methode. Nach vollendeter Inkubation mit den mindestens zwei Peptidasen werde Proben entnommen und homogenisiert. 0,5 g - 2,5 g der homogenisierten Probe werden auf 0,1 mg genau in ein Falcon-Röhrchen eingewogen. 1 ml Norleucin-Lösung (0,1312g/200 ml) werden als interner Standard (IS) zugesetzt und mit 3%iger 5-Sulfosalicylsäure auf 25 ml aufgefüllt. Die Röhrchen werden in den Schüttler eingesetzt und 20 Minuten kräftig geschüttelt. Anschließend wird bei 5000 U/min 5 Minuten zentrifugiert. Der Überstand wird über einen Spritzenfilter (0,45 µm) filtriert. 0,5 ml des Filtrats und 0,5 ml Probenpuffer für die Chromatographie werden in ein Reaktionsgefäß pipettiert und dieses verschlossen. Die so hergestellte Lösung wird zur chromatographischen Analyse am Aminosäureanalysator eingesetzt.

Parallel hierzu wird die Aminosäurenzusammensetzung der Fraktion enthaltend freie Aminosäuren und Oligopeptide (wasserlösliche NPN Fraktion der Zusammensetzung) bestimmt. Hierzu werden wieder 0,5 g - 2,5 g der homogenisierten Probe auf 0,1 mg genau in ein Falcon-Röhrchen eingewogen. 2,5 ml Norleucin-Lösung werden als interner Standard (IS) hinzupipettiert und mit 3%iger 5-Sulfosalicylsäure auf 25 ml aufgefüllt. Die Röhrchen werden wie oben beschrieben geschüttelt und zentrifugiert, die im Niederschlag enthaltenen, wasser-unlöslichen Polypeptide und Proteine werden verworfen. 1 ml des Überstandes wird in ein 50 ml Glasaufschlussgefäß pipettiert, mit 9 ml 6N Salzsäure versetzt und die Gefäße mit einem Schraubdeckel gut verschlossen. Die Gefäße werden in den Heizblock eingesetzt und die Probelösung 8h bei 105°C aufgeschlossen. 2 ml der aufgeschlossenen Lösung werden bis zur Trockene eingeengt. Der Rückstand wird in 1 ml Probenpuffer für die Chromatographie aufgenommen und in ein Reaktionsgefäß überführt, das verschlossen wird. Die so hergestellte Lösung wird zur chromatographischen Analyse am Aminosäureanalysator eingesetzt. Durch Subtraktion des im Schritt 1 ermittelten Anteils an freien Aminosäuren kann zudem der Anteil an Oligopeptiden in der Zusammensetzung rechnerisch ermittelt werden.

Vorzugsweise sind etwa 40 bis 80 Gewichtsprozent, besonders bevorzugt etwa 50 bis 70 Gewichtsprozent der freien Aminosäuren essentielle Aminosäuren, also Isoleucin, Leucin, Lysin, Methionin, Phenylalanin, Threonin, Tryptophan und Valin.

Vorzugsweise sind alle 20 proteinogenen Aminosäuren, also die Aminosäuren Isoleucin, Leucin, Lysin, Methionin, Phenylalanin, Threonin, Tryptophan, Valin, Alanin, Arginin, Asparaginsäure, Asparagin, Cystein, Glutamin, Glutaminsäure, Glycin, Histidin, Prolin, Serin und Tyrosin, in der durch das erfindungsgemäße Herstellungsverfahren erzeugten Zusammensetzung enthalten.

Das gleichzeitige Vorhandensein von freien Aminosäuren, Oligopeptiden und Polypeptiden in den genannten Konzentrationen und den sich daraus ergebenden Verhältnissen zueinander in der hergestellten Zusammensetzung bietet den Vorteil der stufenweisen, lückenlosen Versorgung mit Aminosäuren, praktisch direkt nach Aufnahme der Zusammensetzung über mehrere Stunden hinweg.

Erfindungsgemäß sind die mindestens zwei verschiedenen Peptidasen mikrobiellen Ursprungs. Gemäß einer bevorzugten Weiterbildung sind die mindestens zwei verschiedenen Peptidasen bakteriellen oder fungalen Ursprungs. Mikrobielle Peptidasen sind im Vergleich zu menschlichen oder Säuger Peptidasen deutlich vielfältiger. Dies bietet den Vorteil, dass auch für den Menschen schwer zu verwertende Eiweißquellen effizient verdaut werden können.

Unter dem Begriff "mikrobiellen Ursprungs" sind Peptidasen zu verstehen, deren Aminosäuresequenz jener einer mikrobiellen Peptidase, also einer Peptidase welche in einem Mikroorganismus endogen exprimiert wird, entspricht. Der Organismus, in welchem die Peptidase zum Beispiel durch heterologe Expression hergestellt wurde, ist für die Bezeichnung des Ursprungs im Sinne der Erfindung nicht relevant. Peptidasen mikrobiellen Ursprungs werden somit endogen in Mikroorganismen exprimiert. Als Mikroorganismen im Sinne der Erfindung ist die Gesamtheit aller nicht mit bloßem Auge erkennbaren Organismen zu verstehen. Dazu gehören Viren, Bakterien, Archaeen, Protozoen, Pilze und Mikro-Algen.

Gemäß einer bevorzugten Weiterbildung weist die Zusammensetzung aufweisend mindestens ein Protein eine Proteinkonzentration von 10 bis 20 Gewichtsprozent bezogen auf das Gesamtgewicht der Zusammensetzung aufweisend mindestens ein Protein auf. Es wurde gefunden, dass die genannten Substratkonzentrationen für die Enzymaktivität vorteilhaft sind, da Enzym und Substrat bei den genannten Substratkonzentrationen vergleichsweise schnell in Kontakt treten können. Diese Weiterbildung bietet somit den Vorteil guter Ausbeuten an freien Aminosäuren und/oder Oligopeptiden und/oder einer kurzen Verfahrensdauer.

Gemäß einer bevorzugten Weiterbildung wird die Zusammensetzung aufweisend mindestens ein Protein für mindestens 1 h, vorzugsweise für eine Zeitspanne von 1 h bis 24 h, besonders bevorzugt für eine Zeitspanne von 4 h bis 12 h, am meisten bevorzugt für eine Zeitspanne von 6 h bis 10 h mit zumindest einer der mindestens zwei verschiedenen Peptidasen inkubiert. Diese Weiterbildung bietet den Vorteil, dass sowohl der Anspruch einer effizienten Aufspaltung des mindestens einen Ausgangsproteins als auch jener an einen zeiteffizienten Prozess erfüllt wird. Durch die genannten Inkubationszeiten kann ein optimales Verhältnis an freien Aminosäuren, Oligopeptiden und Polypeptiden erhalten werden.

Gemäß einer bevorzugten Weiterbildung wird die Zusammensetzung aufweisend mindestens ein Protein bei einer Temperatur von 45°C bis 55°C mit den mindestens zwei verschiedenen Peptidasen inkubiert. Diese relativ hohen Temperaturen bieten den Vorteil, dass durch diese relativ hohen Temperaturen eine Kontamination mit Bakterien, und in Folge auch eine Kontamination mit Bakteriophagen, vermieden werden kann. Dadurch werden Gesundheitsrisiken minimiert und Fäulungsprozesse, durch welche Geschmack und Geruch des Endprodukts negativ beeinflusst werden können, verhindert. Die Erkenntnis, dass eine enzymatische Hydrolyse von Proteinen bei solch relativ hohen Temperaturen durchführbar ist, war vollkommen überraschend, da Enzymhersteller üblicherweise Inkubationstemperaturen von etwa 37°C für Peptidasen empfehlen, um die Enzymdenaturierung bei höheren Temperaturen zu vermeiden. Es wurde gefunden, dass bei Temperaturen von etwa 50°C die verwendeten Peptidasen besonders hohe Aktivitäten zeigen und der Herstellungsprozess dadurch verkürzt werden kann. Um optimale Ausbeuten an freien Aminosäuren und Oligopeptiden zu erhalten, kann es sinnvoll sein, die mindestens zwei Peptidasen in höheren Mengen als üblicherweise für enzymatischen Verdau bei 37°C empfohlen einzusetzen. Außerdem kann sich die wiederholte Zugabe der Peptidasen während des Herstellungsprozesses positiv auf die Ausbeute an freien Aminosäuren und Oligopeptiden auswirken.

Gemäß einer bevorzugten Weiterbildung hat die Zusammensetzung aufweisend mindestens ein Protein bei Zugabe der mindestens zwei verschiedenen Peptidasen einen pH-Wert von 4 bis 6, bevorzugt von 4,5 bis 5,5, wobei der pH-Wert gegebenenfalls durch die Zugabe mindestens einer organischen Säure, insbesondere ausgewählt aus Milchsäure, Zitronensäure und Äpfelsäure eingestellt wird. Vorzugsweise werden keine Bakterien oder sonstige Mikroorganismen wie zum Beispiel Hefen während des Herstellungsprozesses zugesetzt. Die genannten pH-Wert Bereiche sind für die Enzymaktivität von Peptidasen optimal. Bei der konventionellen Fermentation mit Peptidase-produzierenden Bakterien muss der pH-Wert hingegen auf etwa 3 bis 4 abgesenkt werden. Das erzeugte fermentierte Produkt hat dadurch einen sehr sauren Geschmack. Durch die Verwendung aufgereinigte Peptidasen in einem pH Bereich von über 4 kann der Geschmack des erzeugten Produkts deutlich verbessert werden. Die erzeugten Zusammensetzungen weisen eine leichte sensorische Säure bis hin zu Neutralgeschmack auf.

Gemäß einer bevorzugten Weiterbildung weist das Verfahren zusätzlich den Schritt der Zugabe von Calciumcarbonat und/oder Magnesiumcarbonat auf, wobei die Zugabe vorzugsweise nach Zugabe der mindestens zwei verschiedenen Peptidasen, besonders bevorzugt nach Abschluss der Inkubation der Zusammensetzung aufweisend mindestens ein Protein mit den mindestens zwei Peptidasen, erfolgt. Diese Weiterbildung bietet den Vorteil, dass das Verhältnis von Calcium zu Magnesium im Endprodukt auf einen gewünschten Wert eingestellt werden kann. Vorzugsweise liegt das Gewichtsverhältnis von Magnesium zu Calcium in der erzeugten Zusammensetzung von etwa 1,25:1 bis etwa 3:1, besonders bevorzugt von etwa 1,5:1 bis etwa 2,5:1 und am meisten bevorzugt bei etwa 2:1. Diese Weiterbildung bietet des Weiteren den Vorteil, dass der pH-Wert des Endprodukts nach Abschluss der enzymatischen Hydrolyse erhöht werden kann, wodurch der Geschmack des Endprodukts verbessert werden kann.

Gemäß einer bevorzugten Weiterbildung weist die Zusammensetzung aufweisend mindestens ein Protein mindestens eine Eiweißquelle ausgewählt aus Molke, Molkeprotein-Konzentrat, Milchprotein-Konzentrat, Schlämpe aus der Lebensmittelproduktion wie zum Bespiel Destillerie-Schlämpe oder Brauerei-Schlämpe, Erbsen, Linsen, Bohnen, Soja und eine Kombination aus zwei oder mehreren hiervon auf. Die Verwendung natürlicher Eiweißquellen, wie der oben genannten, bietet den Vorteil, dass die in diesen Eiweißquellen natürlicherweise vorkommenden Inhaltsstoffe wie Co-Enzyme, Vitamine und Spurenelemente in der erzeugten Zusammensetzung erhalten bleiben und eine besonders rasche und/oder effiziente Resorption im Darm bewirken können. Prinzipiell sind alle Arten von Eiweißquellen als Substrat für den erfindungsgemäßen Herstellungsprozess denkbar. Je nach Verwendung des Endprodukts können Eiweißquellen mit besonders hoher Wertigkeit wie zum Beispiel Molke und Molkeprotein-Konzentrat, pflanzliche Eiweißquellen wie zum Beispiel Soja, Erbsen, Linsen, Bohnen, Brauerei- und Destillerie-Schlämpen zur Herstellung vegetarischer oder veganer Produkte, oder relativ kostengünstige Eiweißquellen wie Brauerei und Destillerie-Schlämpen gewählt und gegebenenfalls kombiniert werden. Molke als Eiweißquelle bietet neben der hohen Wertigkeit des Weiteren den Vorteil eines hohen Anteils an zusätzlichen, für den Menschen wertvollen Inhaltsstoffen, wie Vitaminen, Co-Enzymen, Mineralstoffen und anderen. Diese "Molkematrix" steigert zudem die Bioverfügbarkeit der Aminosäuren, Oligopeptide und Polypeptide durch eine beschleunigte Resorption. Die Versorgung mit Aminosäuren und Oligopeptiden, welche in einer solchen Molkematrix vorliegen, wird als effizienter erachtet, als die Versorgung mit chemisch synthetisierten Aminosäuren, die einem Produkt zugesetzt werden. Molkeprotein-Konzentrat bietet zudem den Vorteil eines hohen Eiweißanteils von etwa 80% und eines geringen Kohlenhydrat- und Fettanteils. Somit ist Molkeprotein-Konzentrat vergleichsweise kalorienarm. Darüber hinaus hat es einen sehr geringen Lactoseanteil. Unabhängig von der eingesetzten Eiweißquelle kann die im Endprodukt verbleibende Lactose gegebenenfalls unproblematisch entfernt werden, zum Beispiel durch die Zugabe aufgereinigter Lactase oder Lactase-produzierender Lactobazillen.

Unter dem Begriff "Eiweißquelle" im Sinne der Erfindung ist eine Zusammensetzung aufweisend mindestens ein Protein, vorzugsweise ein Proteingemisch, zu verstehen. Die Begriffe "Eiweiß" und "Protein" werden in diesem Kontext synonym verwendet und umfassen tierische, pflanzliche und mikrobielle Proteine.

Gemäß einer bevorzugten Weiterbildung beträgt die Gesamtmenge an zugegebener Peptidase jeweils von 500 bis 20.000, vorzugsweise von 1.000 bis 15.000, besonders bevorzugt von 2.000 bis 10.000 U pro 100 Gramm des mindestens einen Proteins in der Zusammensetzung aufweisend mindestens ein Protein.

Unter einem U im Sinne der Erfindung ist jene Enzymmenge zu verstehen, die ein Mikromol Substratprotein pro Minute bei 37°C spaltet.

Erfindungsgemäß beträgt die Gesamtmenge an zugegebener Peptidase von 2 bis 10 Gramm pro 100 Gramm des mindestens einen Proteins in der Zusammensetzung aufweisend mindestens ein Protein.

Die angegebenen Mengen an zugegebener Peptidase liegen über den Herstellerempfehlungen für einen enzymatischen Verdau bei 37°C. Diese Weiterbildungen bieten den Vorteil, dass die Effizienz des enzymatischen Verdaus des mindestens einen Ausgangsproteins gesteigert werden kann, dass also die Ausbeute an freien Aminosäuren und/oder Oligopeptiden erhöht werden kann und/oder dass die Dauer des Herstellungsprozesses verringert werden kann. Es wurde überraschend gefunden, dass der Einsatz der Peptidasen im Überschuss, zum Beispiel in den angegebenen Mengenbereichen, eine Erhöhung der Inkubationstemperatur auf über 40°C bis zu etwa 60°C erlaubt, ohne dass die enzymatische Hydrolyse durch die Enzymdegradation vorzeitig zum Erliegen käme. Durch die Erhöhung der Inkubationstemperatur auf über 40°C wird wiederum das Risiko einer Kontamination mit Bakterien, inklusive bakterieller Krankheitserreger, und damit gleichzeitig das Risiko einer Kontamination mit Bakteriophagen verringert oder ganz vermieden. Vorzugsweise wird der Herstellungsprozess bei etwa 45°C bis etwa 55°C, besonders bevorzugt bei etwa 50°C durchgeführt, da in diesem Temperaturbereich eine Kontamination mit Bakterien und/oder Bakteriophagen weitestgehend verhindert werden kann und die Degradation der zugesetzten Peptidasen gleichzeitig minimiert wird.

Gemäß einer bevorzugten Weiterbildung erfolgt eine wiederholte Zugabe von mindestens einer der mindestens zwei Peptidasen zu der Zusammensetzung aufweisend mindestens ein Protein. Diese Weiterbildung bietet den Vorteil, dass das vorzeitige Erliegen des Prozesses durch die Degradation der zugesetzten Peptidasen vermieden werden kann, beziehungsweise, dass die Gesamtmenge an zugesetzter Peptidase, insbesondere bei Inkubationstemperaturen von über 40°C, über 45°C oder bei etwa 50°C, verringert werden kann.

Gemäß einer bevorzugten Weiterbildung wird mindestens eine der mindestens zwei Peptidasen wiederholt in im Wesentlichen gleich großen Mengen und in im Wesentlichen gleich großen zeitlichen Abständen zu der Zusammensetzung aufweisend mindestens ein Protein zugegeben. Es wurde gefunden, dass sich durch die wiederholte Zugabe der Peptidasen in im Wesentlichen gleich großen Mengen und in im Wesentlichen gleich großen zeitlichen Abständen die Effizienz der enzymatischen Hydrolyse gesteigert werden kann, bzw. dass bei vergleichbarer Zusammensetzung des erhaltenen Endprodukts die Gesamtmenge an eingesetzten Peptidasen verringert werden kann.

Gemäß einer bevorzugten Weiterbildung erfolgt die Zugabe der mindestens einen Endopeptidase vor der Zugabe der mindestens einen Exopeptidase. Überraschender Weise wurde gefunden, dass dadurch der Bittergeschmack der erzeugten Zusammensetzung verringert oder sogar ganz vermieden werden kann. Insbesondere die Aminosäuren Valin, Leucin, Isoleucin und Arginin haben einen bitteren Geschmack. Eine hohe Konzentration dieser freien Aminosäuren kann eine Zusammensetzung sehr bitter bis ungenießbar machen. Aminosäure-haltige Zusammensetzungen, welche durch bakterielle Fermentation, also durch die Zugabe Peptidase-produzierender Bakterien erhalten werden, haben typischerweise einen ausgeprägten Bittergeschmack, der sie für den menschlichen Verzehr ungeeignet machen kann. Die Verringerung der Bitterkeit der durch das erfindungsgemäße Verfahren erzeugten Zusammensetzung könnte durch die Bildung von Ornithin während des enzymatischen Verdaus bewirkt werden. Es ist bekannt, dass Ornithin zur Verringerung der Bitterkeit von Aminosäure-haltigen Zusammensetzungen beitragen kann.

Gemäß einer bevorzugten Weiterbildung wird die Zusammensetzung aufweisend mindestens ein Protein zuerst für 1 bis 10 h, vorzugsweise für 2 bis 6 h, besonders bevorzugt für etwa 5 h mit der mindestens einen Endopeptidase und anschließend für 3 bis 40 h, vorzugsweise für 20 bis 40 h, besonders bevorzugt für etwa 30 h mit der mindestens einen Exopeptidase inkubiert, wobei die Inkubation mit der Exopeptidase vorzugsweise in Gegenwart der mindestens einen Endopeptidase erfolgt. Vorzugsweise wird die mindestens eine Endopeptidase und/oder die mindestens eine Exopeptidase wiederholt zugesetzt, vorzugsweise zweimal, dreimal, viermal, fünfmal oder sechsmal in im Wesentlichen gleich großen Mengen und in im Wesentlichen gleich großen zeitlichen Abständen. Diese Weiterbildung bietet den Vorteil, dass eine Zusammensetzung mit optimalen Mengenverhältnissen von freien Aminosäuren, Oligopeptiden und Polypeptiden erhalten wird.

Gemäß einem zweiten Aspekt betrifft die Erfindung eine Zusammensetzung aufweisend freie Aminosäuren, Oligopeptide und Polypeptide, welche durch mindestens eines der hierin beschriebenen Verfahren erhältlich ist, dadurch gekennzeichnet, dass die Zusammensetzung (a) von 0,5 Gewichtsprozent bis 25 Gewichtsprozent an freien Aminosäuren bezogen auf das Gesamtgewicht von freien Aminosäuren, Oligopeptiden, Polypeptiden und Proteinen ohne die zugesetzten Peptidasen aufweist und (b) von 25 bis 65 Gewichtsprozent an Oligopeptiden bezogen auf das Gesamtgewicht von freien Aminosäuren, Oligopeptiden, Polypeptiden und Proteinen ohne die zugesetzten Peptidasen aufweist.

Beschrieben wird außerdem eine Zusammensetzung aufweisend freie Aminosäuren, Oligopeptide, Polypeptide, Coenzyme, Vitamine und Spurenelemente, wobei die Zusammensetzung vorzugsweise zumindest die folgenden freien Aminosäuren aufweist: Isoleucin, Leucin, Lysin, Methionin, Phenylalanin, Threonin, Tryptophan und Valin, besonders bevorzugt wobei die Zusammensetzung die 20 proteinogenen Aminosäuren Isoleucin, Leucin, Lysin, Methionin, Phenylalanin, Threonin, Tryptophan, Valin, Alanin, Arginin, Asparaginsäure, Asparagin, Cystein, Glutamin, Glutaminsäure, Glycin, Histidin, Prolin, Serin und Tyrosin aufweist.

Die freien Aminosäuren, Oligopeptide und Polypeptide wurden durch enzymatische Hydrolyse mindestens eines Proteins mit mindestens zwei verschiedenen aufgereinigten Peptidasen erhalten. Dies bietet gegenüber herkömmlicher Fermentation von Eiweißquellen durch Peptidase-produzierende Bakterien oder sonstige Mikroorganismen den Vorteil besonders hoher Anteile an freien Aminosäuren und/oder Oligopeptiden in der Zusammensetzung. insbesondere kann bei entsprechender Gestaltung der enzymatischen Hydrolyse mit den mindestens zwei verschiedenen aufgereinigten Peptidasen eine Zusammensetzung mit allen acht für den Menschen essentiellen Aminosäuren oder sogar mit allen zwanzig proteinogenen Aminosäuren erhalten werden. Diese Zusammensetzungen eignen sich daher, Versorgungslücken an einzelnen Aminosäuren zu schließen und können so Mangelerkrankungen, Marasmus und einer Schwächung des Immunsystems vorbeugen.

Erfindungsgemäß weist die Zusammensetzung von 0,5 Gewichtsprozent bis 25 Gewichtsprozent, vorzugsweise von 2 Gewichtsprozent bis 20 Gewichtsprozent, besonders bevorzugt von 5 Gewichtsprozent bis 15 Gewichtsprozent an freien Aminosäuren bezogen auf das Gesamtgewicht von freien Aminosäuren, Oligopeptiden, Polypeptiden und Proteinen ohne die zugesetzten Peptidasen auf.

Gemäß einer weiteren bevorzugten Weiterbildung weist die Zusammensetzung von 0,3 bis 15 Gewichtsprozent, vorzugsweise von 1,2 Gewichtsprozent bis 12 Gewichtsprozent, besonders bevorzugt von 3 bis 9 Gewichtsprozent an den freien essentiellen Aminosäuren Isoleucin, Leucin, Lysin, Methionin, Phenylalanin, Threonin, Tryptophan und Valin in Summe bezogen auf das Gesamtgewicht von freien Aminosäuren, Oligopeptiden, Polypeptiden und Proteinen ohne die zugesetzten Peptidasen auf.

Erfindungsgemäß weist die Zusammensetzung von 25 bis 65 Gewichtsprozent an Oligopeptiden bezogen auf das Gesamtgewicht von freien Aminosäuren, Oligopeptiden, Polypeptiden und Proteinen ohne die zugesetzten Peptidasen auf.

Gemäß einer weiteren bevorzugten Weiterbildung liegen 0 Gewichtsprozent bis 40 Gewichtsprozent, vorzugsweise 0 Gewichtsprozent bis 20 Gewichtsprozent, besonders bevorzugt 0 Gewichtsprozent bis 10 Gewichtsprozent des mindestens einen Ausgangsproteins für die enzymatische Hydrolyse in der Zusammensetzung als ungespaltenes Protein vor.

Diese Weiterbildungen bieten den Vorteil der stufenweisen, lückenlosen Versorgung mit Aminosäuren, praktisch direkt nach Aufnahme der Zusammensetzung über mehrere Stunden hinweg.

Gemäß einer bevorzugten Weiterbildung weist die Zusammensetzung einen pH-Wert von 4 bis 6, besonders bevorzugt von 4,5 bis 5,5 auf. Diese Weiterbildung bietet den Vorteil, dass der pH-Wert der Zusammensetzung jenem der Haut ähnelt und daher vorteilhaft für die Herstellung von Körperpflegeprodukten verwendet werden kann. Diese Weiterbildung bietet des Weiteren den Vorteil, dass sie relativ geschmacksneutral, insbesondere nicht zu sauer ist, und daher vorteilhaft für die Herstellung von Nahrungsmitteln verwendet werden kann.

Gemäß einer bevorzugten Weiterbildung beträgt das Gewichtsverhältnis von Magnesium zu Calcium in der Zusammensetzung von 1,25:1 bis 3:1, vorzugsweise von 1,5:1 bis 2,5:1, besonders bevorzugt etwa 2:1.

Die Zusammensetzung kann direkt nach dem enzymatischen Verdau ohne weitere Aufreinigungs- und/oder Isolierungsschritte zur Herstellung von Nahrungsmitteln oder Körperpflegeprodukten weiterverwendet werden. Auch die Entfernung einzelner Komponenten der Zusammensetzung, wie zum Beispiel Lactose oder ungespaltenem Protein und/oder wasserunlöslicher Polypeptide ist denkbar. Das Volumen der durch den enzymatischen Verdau erhaltenen Zusammensetzung kann auch erst durch beliebige Maßnahmen eingeengt werden oder es kann ein Pulver aufweisend Aminosäuren und Oligopepetide sowie gegebenenfalls Polypeptide und/oder ungespaltenes Protein aus der erzeugten Zusammensetzung gewonnen werden.

Gemäß einem dritten Aspekt betrifft die Erfindung die Verwendung der erfindungsgemäßen Zusammensetzung zur Herstellung eines Nahrungsmittels, insbesondere zur Herstellung eines Fruchtsaftgetränks, eines Milchgetränks, eines Molkegetränks, eines Joghurts, eines Ayrans oder eines Kefirs.

Gemäß einer bevorzugten Weiterbildung wird ein Teil des Wassers eines Nahrungsmittels, insbesondere eines vorbekannten Nahrungsmittels, durch die erfindungsgemäße Zusammensetzung ersetzt. Somit lassen sich Nahrungsmittel besonders einfach durch die Zugabe der erfindungsgemäßen Zusammensetzung "veredeln" und in ihrer Wertigkeit verbessern.

Gemäß einem vierten Aspekt betrifft die Erfindung die Verwendung der erfindungsgemäßen Zusammensetzung zur Herstellung eines Körperpflegeprodukts, insbesondere zur Herstellung einer Seife, eines Shampoos, einer Haarkur, einer Haarmaske, einer Haarpackung, eines Haarwassers, eines Conditioners, eines Badezusatzes, einer Body Lotion, eines Körpergels, einer Lotion zur Behandlung von Rosacea, einer Creme zur Akne Behandlung, einer Gesichtsmaske oder einer Gesichtscreme.

Gemäß einer bevorzugten Weiterbildung wird ein Teil des Wassers, vorzugsweise mindestens 80% des Wassers, eines Körperpflegeprodukts, insbesondere eines vorbekannten Körperpflegeprodukts, vorzugsweise einer Seife, durch die erfindungsgemäße Zusammensetzung ersetzt. Somit lassen sich Körperpflegeprodukte besonders einfach durch die Zugabe der erfindungsgemäßen Zusammensetzung "veredeln" und in ihrer Wertigkeit verbessern. Hervorzuheben ist hierbei insbesondere die verbesserte Hautverträglichkeit solcher Zusammensetzungen. Die Oligo- und Polypeptide in so hergestellten Seifen und Körperpflegemitteln können die Collagen-Bildung in Haut und Haar begünstigen und somit Hautreizungen entgegenwirken.

Gemäß einem fünften Aspekt betrifft die Erfindung ein Nahrungsmittel aufweisend die erfindungsgemäße Zusammensetzung.

Gemäß einer bevorzugten Weiterbildung beträgt der Gesamtanteil an freien Aminosäuren, Oligopeptiden und Polypeptiden in dem Nahrungsmittel von 2 Gewichtsprozent bis 25 Gewichtsprozent, vorzugsweise etwa 5 bis 10 Gewichtsprozent.

Gemäß einer bevorzugten Weiterbildung beträgt die Gesamtmenge an freien Aminosäuren, Oligopeptiden und Polypeptiden in dem Nahrungsmittel von 5 bis 50 Gramm, vorzugsweise etwa 10 Gramm. Diese Weiterbildung bietet den Vorteil, dass ein signifikanter Anteil der empfohlenen Eiweiß-Tagesdosis durch die Aufnahme des Nahrungsmittels gedeckt werden kann. Empfohlen wird für einen Erwachsenen eine Aufnahme von etwa 50 bis 100 Gramm Protein pro Tag. bei erhöhter körperlicher Belastung verdoppelt sich die empfohlene Proteinmenge.

Gemäß einer bevorzugten Weiterbildung ist das Nahrungsmittel ein Getränk aufweisend Magnesium und Calcium. Vorzugsweise liegt das Verhältnis von Magnesium zu Calcium in dem Getränk von 1,25:1 bis 3:1, besonders bevorzugt bei etwa 2:1.

Gemäß einer bevorzugten Ausgestaltung dieser Weiterbildung ist das Getränk ein Ayran. Vorzugsweise beträgt die Gesamtmenge an freien Aminosäuren, Oligopeptiden, Polypeptiden und Proteinen ohne Peptidasen zwischen 5 und 25 g pro Portion, besonders bevorzugt etwa 10 g pro Portion. Vorzugsweise weist das Ayran Getränk ausschließlich pflanzliche Aminosäuren, Oligopeptide und Polypeptide auf, besonders bevorzugt aus Soja-Protein stammende Aminosäuren, Oligopeptide und Polypeptide. Vorzugsweise ist das Getränk im Wesentlichen frei von Bakterien.

Gemäß einer alternativen Ausgestaltung dieser Weiterbildung weist das Getränk Koffein und/oder Taurin auf. Zusammen mit Koffein und/oder Taurin und im Zusammenspiel mit Calcium und Magnesium versorgt das erfindungsgemäße Getränk den Körper mit wichtigen Nährstoffen, und kann so die körperliche Verfassung von Frühaufstehern, Nachtschwärmern oder -Arbeitern verbessern, die Abwehrkräfte stärken und die Ansteckungsgefahr bei Überanstrengung verringern.

Gemäß einem sechsten Aspekt betrifft die Erfindung ein Körperpflegeprodukt aufweisend die erfindungsgemäße Zusammensetzung.

Gemäß einer bevorzugten Weiterbildung ist das Körperpflegeprodukt ein Pflegebad oder ein Badezusatz. Vorzugsweise ist das Pflegebad oder der Badezusatz ein Molkebad aufweisend enzymatisch fermentierte Molke. Vorzugsweise wird das Pflegebad als Fertigprodukt von 1 bis 25 I angeboten. Vorzugsweise weist das Pflegebad oder der Badezusatz weder Seife noch Shampoo auf. Das Pflegebad oder der Badezusatz reinigt die Haut schonend und vermindert deren Austrocknung. Es kann den Säuremantel der Haut regenerieren und unterstützt die Heilung von Entzündungen und Ekzemen der Haut.

Gemäß einer bevorzugten Weiterbildung ist das Körperpflegeprodukt eine Seife. Vorzugsweise weist die Seife einen pH Wert von 5 bis 7, besonders bevorzugt von etwa 5,5 auf. Dies entspricht in etwa dem pH Wert der Haut und ist somit besonders Haut schonend. Aber auch Seifen im alkalischen Bereich, zum Beispiel mit einem pH von etwa 7 bis 8,5 sind nicht ausgeschlossen. Es wurde gefunden, dass die in der Seife enthaltenen, durch enzymatische Fermentation erzeugten Peptide den negativen Effekt der Seife auf den Säuremantel der Haut vermindern können und die Regeneration der Haut unterstützen können. Seifen aufweisend die durch enzymatische Fermentation hergestellte Aminosäure-haltige Zusammensetzung können Hautaustrocknung und Juckreiz mindern.

Beschrieben werden hierin auch Seifen aufweisend Aminosäuren und Peptide, welche durch enzymatische und/oder bakterielle Fermentation erhalten werden.

### Beispiele

### Beispiel 1:

Molkeprotein-Konzentrat WPC 80 mit einem Trockenmasseanteil von 25% und einem Eiweißanteil von 80% in der Trockenmasse wurde mit Wasser 1:2 verdünnt. Der Eiweißanteil der so hergestellten Lösung betrug 10 Gewichtsprozent, bezogen auf des Gesamtgewicht der hergestellten Lösung.

Der pH Wert dieser Lösung wurde mit Zitronensäure auf 5,5 eingestellt.

Es wurden zwei Testansätze mit jeweils 1 I (100 g Eiweiß) hergestellt. Zu beiden Ansätzen wurden zu Beginn und weitere fünfmal im Abstand von jeweils einer Stunde je 2 g einer Endopeptidase mit etwa 1000 SAPU/g zugesetzt. Die Gesamtmenge an zugesetzter Endopeptidase betrug jeweils 12 g/100 g Ausgangseiweiß.

Die Ansätze wurden bei 50°C inkubiert.

Nach fünf Stunden wurde jeweils eine Probe von 50 ml entnommen (Proben I5 und II5 - Vergleichsbeispiel). Die Ansätze wurden über Nacht weiterhin bei 50°C inkubiert.

Am nächsten wurden zu beiden Ansätzen zusätzlich fünfmal im Abstand von jeweils einer Stunde je 2 g einer Exopeptidase mit etwa 900 CPGU zugesetzt.

Die Gesamtmenge an zugesetzter Exopeptidase betrug jeweils 10 g/100 g Ausgangseiweiß. Die Ansätze wurden weiter bei 50°C inkubiert.

Anschließend wurde wieder jeweils eine Probe von 50 ml entnommen (Proben I9 und II9) und alle vier Proben analysiert.

Die Proben wurden hierzu homogenisiert. 0,5 g - 2,5 g der homogenisierten Proben wurden auf 0,1 mg genau in ein Falcon-Röhrchen eingewogen. 1 ml Norleucin-Lösung (0,1312g/200 ml) wurden als interner Standard (IS) zugesetzt und mit 3%iger 5-Sulfosalicylsäure auf 25 ml aufgefüllt. Die Röhrchen wurden in den Schüttler eingesetzt und 20 Minuten kräftig geschüttelt. Anschließend wurde bei 5000 U/min 5 Minuten zentrifugiert. Der Überstand wurde über einen Spritzenfilter (0,45 µm) filtriert. 0,5 ml des Filtrats und 0,5 ml Probenpuffer für die Chromatographie wurden in ein Reaktionsgefäß pipettiert und dieses verschlossen. Die so hergestellte Lösung wurde zur chromatographischen Analyse am Aminosäureanalysator eingesetzt.

Parallel hierzu wurde die Aminosäurenzusammensetzung der Fraktion enthaltend freie Aminosäuren und Oligopeptide bestimmt. Hierzu wurden wieder 0,5 g - 2,5 g der homogenisierten Probe auf 0,1 mg genau in ein Falcon-Röhrchen eingewogen. 2,5 ml Norleucin-Lösung wurden als interner Standard (IS) hinzupipettiert und mit 3%iger 5-Sulfosalicylsäure auf 25 ml aufgefüllt. Die Röhrchen wurden wie oben beschrieben geschüttelt und zentrifugiert, die im Niederschlag enthaltenen, wasser-unlöslichen Polypeptide und Proteine wurden verworfen. 1 ml des Überstandes wurde in ein 50 ml Glasaufschlussgefäß pipettiert, mit 9 ml 6N Salzsäure versetzt und die Gefäße mit einem Schraubdeckel gut verschlossen. Die Gefäße wurden in den Heizblock eingesetzt und die Probelösung 8 h bei 105°C aufgeschlossen. 2 ml der aufgeschlossenen Lösung wurden bis zur Trockene eingeengt. Der Rückstand wurde in 1 ml Probenpuffer für die Chromatographie aufgenommen und in ein Reaktionsgefäß überführt, das verschlossen wird. Die so hergestellte Lösung wurde zur chromatographischen Analyse am Aminosäureanalysator eingesetzt.

Durch Subtraktion des im Schritt 1 ermittelten Anteils an freien Aminosäuren kann des Weiteren der Anteil an Oligopeptiden in der Zusammensetzung rechnerisch ermittelt werden.

Tabelle 1 zeigt die Zusammensetzung der vier untersuchten Proben.

**Tabelle 1: Probenzusammensetzung**

| **Ergebnisse freie Aminosäuren:** | **Probe I 5** | **Probe II 5** | **Probe I 9** | **Probe II 9** |
|---|---|---|---|---|
| | **mg/kg Versuchsansatz** | | | |
| **essentielle Aminosäuren (proteinogene Aminosäuren):** | | | | |
| Threonin (Thr) | 57 | 56 | 709 | 738 |
| Valin (Val) | 112 | 111 | 977 | 949 |
| Methionin (Met) | 75 | 85 | 135 | 142 |
| Isoleucin (Iso) | 49 | 44 | 344 | 361 |
| Leucin (Leu) | 947 | 853 | >2800 | >2800 |
| Phenylalanin (Phe) | 175 | 143 | 843 | 815 |
| Lysin (Lys) | 1279 | 1209 | >2800 | >2800 |
| Tryptophan (Trp) | nicht bestimmt | | | |

| **semi-essentielle Aminosäuren (proteinogene Aminosäuren)** | | | | |
|---|---|---|---|---|
| Histidin (His) | 74 | 68 | 384 | 384 |
| Arginin (Arg) | 264 | 247 | 795 | 791 |

| **nicht-essentielle Aminosäuren (proteinogene Aminosäuren)** | | | | |
|---|---|---|---|---|
| Asparaginsäure (Asp) | 25 | 23 | 390 | 378 |
| Serin (Ser) | 60 | 57 | 395 | 404 |
| Asparagin (Asn) | 49 | 30 | 343 | 292 |
| Glutaminsäure (Glu) | 308 | 248 | 1591 | 1588 |
| Glycin (Gly) | <10 | <10 | 116 | 124 |
| Alanin (Ala) | 105 | 93 | 634 | 665 |
| Cystein (Cys) | nicht bestimmt | | | |
| Tyrosin (Tyr) | 148 | 142 | 814 | 818 |
| Prolin (Pro) | 29 | 40 | 104 | 125 |
| Glutamin (Gln) | nicht bestimmt | | | |

| **weitere bestimmte Substanzen** | | | | |
|---|---|---|---|---|
| Taurin (Tau) | nicht auswertbar | nicht auswertbar | nicht auswertbar | nicht auswertbar |
| Citrullin (Cit) | <10 | <10 | 34 | 41 |
| Cystin (Cys2) | 186 | 189 | 341 | 348 |
| Cystathionin (Cystha) | 191 | 173 | nicht auswertbar | nicht auswertbar |
| 3-Methyl-Histidin (3Mehis) | <10 | <10 | <10 | <10 |
| 1-Methyl-Histidin (1Mehis) | <10 | <10 | <10 | <10 |
| Hydroxylysin (Hylys) | <10 | <10 | <10 | <10 |
| Ornithin (Orn) | <10 | <10 | <10 | < 10 |
| Hydroxyprolin (Hypro) | <10 | <10 | <10 | <10 |

| **Ergebnisse NPN-Extrakt (Oliqopeptide und freie Aminosäuren):** | **Probe I 5** | **Probe II 5** | **Probe I 9** | **Probe II 9** |
|---|---|---|---|---|
| | **mg/kg Versuchsansatz** | | | |
| **essentielle Aminosäuren (proteinogene Aminosäuren)** | | | | |
| Threonin (Thr) | 4813 | 4641 | 5223 | 5266 |
| Valin (Val) | 2583 | 2672 | 3069 | 3287 |
| Methionin (Met) | 791 | 154 | 533 | 223 |
| Isoleucin (Iso) | 2968 | 2840 | 3410 | 3701 |
| Leucin (Leu) | 5909 | 5696 | 6864 | 6944 |
| Phenylalanin (Phe) | 1590 | 1564 | 1890 | 1846 |
| Lysin (Lys) | 5788 | 5490 | 6544 | 6530 |
| Tryptophan (Trp) | nicht bestimmt | | | |

| **semi-essentielle Aminosäuren (proteinogene Aminosäuren)** | | | | |
|---|---|---|---|---|
| Histidin (His) | 1081 | 1004 | 1239 | 1190 |
| Arginin (Arg) | 1300 | 1185 | 1360 | 1285 |

| **nicht-essentielle Aminosäuren (proteinogene Aminosäuren)** | | | | |
|---|---|---|---|---|
| Asparaginsäure (Asp) | 5459 | 5145 | nicht auswertbar | 2004 |
| Serin (Ser) | 3344 | 2694 | 3394 | 3249 |
| Asparagin (Asn) | nn | nn | nn | nn |
| Glutaminsäure (Glu) | 11235 | 9516 | 9333 | 8550 |
| Glycin (Gly) | 941 | 873 | 1038 | 975 |
| Alanin (Ala) | 3279 | 3123 | 3225 | 3176 |
| Cystein (Cys) | nicht bestimmt | | | |
| Tyrosin (Tyr) | 1122 | 860 | 1563 | 1420 |
| Prolin (Pro) | 4722 | 4722 | 4644 | 4522 |
| Glutamin (Gln) | nicht bestimmt | | | |

| **weitere bestimmte Substanzen** | | | | |
|---|---|---|---|---|
| Taurin (Tau) | nicht auswertbar | nicht auswertbar | nicht auswertbar | nicht auswertbar |
| Citrullin (Cit) | nn | nn | nn | nn |
| Cystin (Cys2) | 747 | 488 | 1374 | 1160 |
| Cystathionin (Cystha) | nn | nn | nn | nn |
| 3-Methyl-Histidin (3Mehis) | nn | nn | nn | nn |
| 1-Methyl-Histidin (1Mehis) | nn | nn | nn | nn |
| Hydroxylysin (Hylys) | nn | nn | nn | nn |
| Ornithin (Orn) | nn | nn | nn | nn |
| Hydroxyprolin (Hypro) | nn | nn | nn | nn |

In den Ansätzen, welche mit der Endo- und der Exopeptidase inkubiert wurden, waren nach Abschluss der Hydrolyse etwa 50 bis 60% Oligopeptide und etwa 15% freie Aminosäuren aufgeteilt auf etwa 10% essentielle Aminosäuren und 5% nicht essentielle Aminosäuren, jeweils bezogen auf das Gesamtgewicht an Ausgangseiweiß, vorliegend 100 g Eiweiß, vorhanden.

Außerdem wurde der pH Wert, der Lactose Gehalt und der Geschmack der vier Proben getestet. Die Ergebnisse sind in Tabelle 2 zusammengefasst.

**Tabelle 2: Geschmacksanalyse der erzeugten Hydrolysate:**

| | **Probe I5** | **Probe II5** | **Probe I9** | **Probe II9** | **WPC 80 1:2** |
|---|---|---|---|---|---|
| pH-Wert | 4,97 | 5,03 | 5,04 | 5,10 | |
| Geschmack | nicht bitter | nicht bitter | nicht bitter | nicht bitter | |
| Lactose [g/100g] | | | | 0,9 | 0,8 |

### Beispiel 2:

Molkeprotein-Konzentrat WPC 80 mit einem Trockenmasseanteil von 25% und einem Eiweißanteil von 80% in der Trockenmasse wurde mit Wasser 1:2 verdünnt. Der Eiweißanteil der so hergestellten Lösung betrug 9,98 Gewichtsprozent, bezogen auf des Gesamtgewicht der hergestellten Lösung.

Der pH Wert dieser Lösung betrug ursprünglich 6,32 und wurde anschließend mit Zitronensäure auf 5,45 eingestellt.

Das Ansatzvolumen betrug jeweils 2 I. Es wurde eine Endopeptidase mit etwa 1000 SAPU/g und eine Exopeptidase mit etwa 900 CPGU zugesetzt. Die enzymatische Hydrolyse erfolgte bei 50°C.

Tabelle 3 zeigt das Hydrolyse-Schema:

**Tabelle 3: Hydrolyse-Schema**

| **Tag** | **Uhrzeit** | **Ansatz EZ 40** | **Ansatz EZ 41** |
|---|---|---|---|
| Tag 1 | 7:05 | 2 g Endopeptidase | 2 g Endopeptidase |
| | 9:05 | 2 g Endopeptidase | 2 g Endopeptidase |
| | | 2g Exopeptidase | 2g Exopeptidase |
| | 11:10 | 2 g Endopeptidase | 2 g Endopeptidase |
| | | 2g Exopeptidase | 2g Exopeptidase |
| | 14:10 | Ende | 4 g Endopeptidase |
| | | | 4g Exopeptidase |
| Tag 2 | 9:00 | | Ende |
| Gesamtmenge Endopeptidase [g/100 g Ausgangsprotein] | | 3g | 5g |
| Gesamtmenge Exopeptidase [g/100 g Ausgangsprotein] | | 2g | 4g |

Anschließend wurden Proben entnommen und wie in Beispiel 1 beschrieben analysiert.

Tabelle 4 zeigt die Zusammensetzung der untersuchten Proben.

**Tabelle 4: Probenzusammensetzung**

| | **Ergebnisse freie Aminosäuren:** | | **Ergebnisse NPN-Extrakt (Oligopeptide und freie Aminosäuren):** | |
|---|---|---|---|---|
| | **EZ40** | **EZ41** | **EZ40** | **EZ41** |
| | **mg/kg Versuchsansatz** | | | |
| **essentielle Aminosäuren (proteinogene Aminosäuren)** | | | | |
| Threonin (Thr) | 143 | 434 | 3782 | 4482 |
| Valin (Val) | 166 | 513 | 2222 | 2694 |
| Methionin (Met) | 114 | 257 | 871 | 978 |
| Isoleucin (Iso) | 101 | 283 | 2458 | 2956 |
| Leucin (Leu) | 808 | <2000* | 4643 | >5500* |
| Phenylalanin (Phe) | 143 | 354 | 1217 | 1486 |
| Lysin (Lys) | 1147 | <2000* | <4000* | >4000* |
| Tryptophan (Trp) | nicht bestimmt | | | |

| **semi-essentielle Aminosäuren (proteinogene Aminosäuren)** | | | | |
|---|---|---|---|---|
| Histidin (His) | 124 | 253 | 727 | 949 |
| Arginin (Arg) | 256 | 536 | 956 | 1214 |

| **nicht-essentielle Aminosäuren (proteinogene Aminosäuren)** | | | | |
|---|---|---|---|---|
| Asparaginsäure (Asp) | 46 | 163 | 4136 | 5076 |
| Serin (Ser) | 125 | 289 | 2557 | 2957 |
| Asparagin (Asn) | 61 | 138 | <50 | <50 |
| Glutaminsäure (Glu) | 261 | 666 | >8000* | >8000* |
| Glycin (Gly) | 19 | 48 | 685 | 878 |
| Alanin (Ala) | 124 | 381 | 2637 | >3000* |
| Cystein (Cys) | nicht bestimmt | | | |
| Tyrosin (Tyr) | 193 | 515 | 1064 | 1470 |
| Prolin (Pro) | 21 | 79 | >4000* | >4000* |
| Glutamin (Gln) | nicht bestimmt | | | |

| **weitere bestimmte Substanzen** | | | | |
|---|---|---|---|---|
| Taurin (Tau) | 68 | 83 | 87 | 90 |
| Citrullin (Cit) | <10 | <10 | <50 | <50 |
| Cystin (Cys2) | 103 | 261 | 399 | 516 |
| Cystathionin (Cystha) | <10 | <10 | <50 | <50 |
| 3-Methyl-Histidin (3Mehis) | <10 | <10 | <50 | <50 |
| 1-Methyl-Histidin (1Mehis) | <10 | <10 | <50 | <50 |
| Hydroxylysin (Hylys) | <10 | <10 | <50 | <50 |
| Ornithin (Orn) | <10 | <10 | <50 | <50 |
| Hydroxyprolin (Hypro) | <10 | <10 | <50 | <50 |

Außerdem wurden die Proben hinsichtlich Phasentrennung, pH Wert und Geschmack untersucht. Die Ergebnisse sind in Tabelle 5 zusammengefasst.

**Tabelle 5: Geschmacksanalyse der erzeugten Hydrolysate:**

| | **Probe EZ 40** | **Probe EZ 41** |
|---|---|---|
| Phasentrennung | nein | ja |
| pH Wert | 4,50 | 4,47 |
| Geschmack | bitter | bitter |

### Beispiel 3:

Molkeprotein-Konzentrat WPC 80 mit einem Trockenmasseanteil von 25% und einem Eiweißanteil von 80% in der Trockenmasse wurde mit Wasser verdünnt oder unverdünnt eingesetzt.

Tabelle 6 zeigt die Zusammensetzung der Versuchsansätze mit ihren pH Werten. Eine Einstellung des pHs war nicht erforderlich.

**Tabelle 6: Zusammensetzung Versuchsansätze:**

| | **EZ 42** | **EZ 43** | **EZ 44** | **EZ45** |
|---|---|---|---|---|
| Eiweißgehalt [%] | 11,27 | 20,43 | 11,27 | 10,00 |
| pH Wert | 5,33 | 5,34 | 5,33 | 5,60 |
| Ansatzvolumen [I] | 2 | 2 | 2 | 1 |

Es wurde eine Endopeptidase mit etwa 1000 SAPU/g und eine Exopeptidase mit etwa 900 CPGU zugesetzt. Die enzymatische Hydrolyse erfolgte bei 50°C.

Tabelle 7 zeigt das Hydrolyse-Schema.

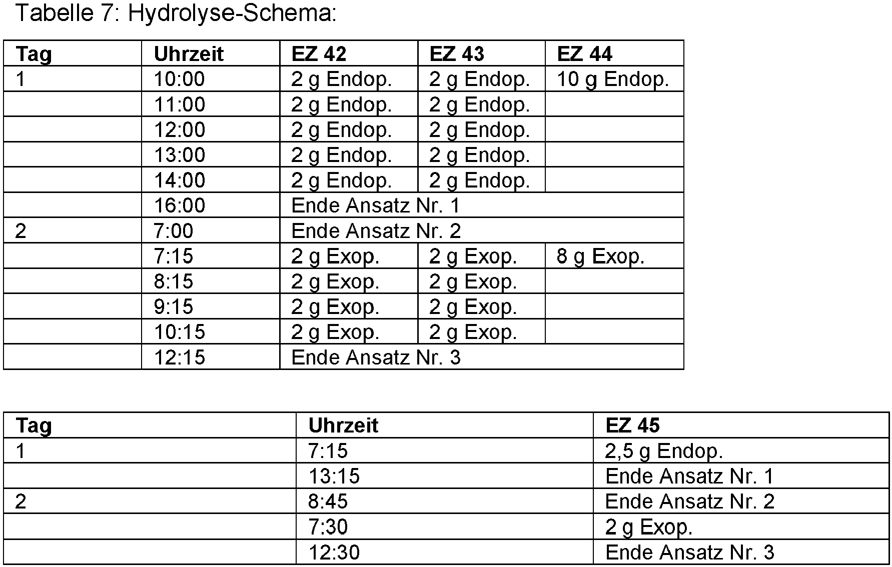

Anschließend wurden Proben genommen und wie in Beispiel 1 beschrieben analysiert.

Tabelle 8 zeigt die Zusammensetzung der untersuchten Proben.

**Tabelle 8: Probenzusammensetzung**

| **Ergebnisse** | **EZ 42** | | | **EZ 43** | | |
|---|---|---|---|---|---|---|
| **freie Aminosäuren:** | **6h** | **21h** | **26h** | **6h** | **21h** | **26h** |
| | mg/kg Versuchsansatz | | | | | |

| **essentielle Aminosäuren (proteinogene Aminosäuren)** | | | | | | |
|---|---|---|---|---|---|---|
| Threonin (Thr) | 85 | 267 | 671 | 71 | 263 | 992 |
| Valin (Val) | 60 | 205 | 631 | 96 | 229 | 818 |
| Methionin (Met) | 150 | 341 | 453 | 203 | 415 | 632 |
| Isoleucin (Iso) | 39 | 156 | 398 | 70 | 186 | 465 |
| Leucin (Leu) | 522 | 1351 | 2536 | 582 | 1407 | 3049 |
| Phenylalanin (Phe) | 126 | 335 | 709 | 165 | 354 | 886 |
| Lysin (Lys) | 1102 | 1731 | 2898 | 1598 | 2126 | 3723 |
| Tryptophan (Trp) | nicht bestimmt | | | nicht bestimmt | | |

| **semi-essentielle Aminosäuren (proteinogene Aminosäuren)** | | | | | | |
|---|---|---|---|---|---|---|
| Histidin (His) | 68 | 164 | 303 | 74 | 150 | 400 |
| Arginin (Arg) | 230 | 408 | 421 | 220 | 448 | 359 |

| **nicht-essentielle Aminosäuren (proteinogene Aminosäuren)** | | | | | | |
|---|---|---|---|---|---|---|
| Asparaginsäure (Asp) | 33 | 134 | 464 | 40 | 121 | 574 |
| Serin (Ser) | 69 | 169 | 368 | 70 | 170 | 613 |
| Asparagin (Asn) | nn | 164 | 386 | 39 | 99 | 609 |
| Glutaminsäure (Glu) | 141 | 614 | 1909 | 194 | 553 | 2305 |
| Glycin (Gly) | 26 | 63 | 133 | 30 | 67 | 195 |
| Alanin (Ala) | 85 | 239 | 554 | 112 | 248 | 751 |
| Cystein (Cys) | nicht bestimmt | | | nicht bestimmt | | |
| Tyrosin (Tyr) | 64 | 260 | 626 | 58 | 251 | 681 |
| Prolin (Pro) | nn | 76 | 150 | nn | 72 | 236 |
| Glutamin (Gln) | nicht bestimmt | | | nicht bestimmt | | |

| **weitere bestimmte Substanzen** | | | | | | |
|---|---|---|---|---|---|---|
| Taurin (Tau) | nicht auswertbar | nicht auswertbar | nicht auswertbar | nicht auswertbar | nicht auswertbar | nicht auswertbar |
| Citrullin (Cit) | nn | nn | 156 | nn | nn | 431 |
| Cystin (Cys2) | nn | 62 | 150 | 18 | 49 | 57 |
| Cystathionin (Cystha) | 89 | 151 | 68 | 115 | 145 | 63 |
| 3-Methyl-Histidin (3Mehis) | nn | nn | nn | nn | nn | nn |
| 1-Methyl-Histidin (1Mehis) | nn | nn | nn | nn | nn | nn |
| Hydroxylysin (Hylys) | nn | nn | nn | nn | nn | nn |
| Ornithin (Orn) | nn | 19 | 55 | nn | nn | 96 |
| Hydroxyprolin (Hypro) | nn | nn | nn | nn | nn | nn |
| | | | | | | |
| Summe | 2889 | 6909 | 14039 | 3755 | 7353 | 17935 |

| **Ergebnisse NPN-Extrakt** | **EZ 42** | | | **EZ 43** | | |
|---|---|---|---|---|---|---|
| **(Oliqopeptide und freie Aminosäuren):** | **6h** | **21h** | **26h** | **6h** | **21h** | **26h** |
| | mg/kg Versuchsansatz | | | | | |

| **essentielle Aminosäuren (proteinogene Aminosäuren)** | | | | | | |
|---|---|---|---|---|---|---|
| Threonin (Thr) | 4151 | 5074 | 4963 | 5574 | 5355 | 1927 |
| Valin (Val) | 4016 | 3634 | 3687 | 5923 | 5821 | 5951 |
| Methionin (Met) | 423 | 768 | 755 | 1098 | 1073 | 307 |
| Isoleucin (Iso) | 3953 | 3864 | 4059 | 6293 | 6110 | 6356 |
| Leucin (Leu) | 8149 | 7809 | 7971 | 12565 | 12778 | 13637 |
| Phenylalanin (Phe) | 2316 | 2175 | 2261 | 3186 | 3285 | 3533 |
| Lysin (Lys) | 7261 | 6975 | 7228 | 10794 | 10943 | 12236 |
| Tryptophan (Trp) | nicht bestimmt | | | nicht bestimmt | | |

| **semi-essentielle Aminosäuren (proteinogene Aminosäuren)** | | | | | | |
|---|---|---|---|---|---|---|
| Histidin (His) | 1536 | 1327 | 1544 | 2057 | 2103 | 2547 |
| Arginin (Arg) | 2308 | 1487 | 1321 | 2253 | 2599 | 1666 |

| **nicht-essentielle Aminosäuren (proteinogene Aminosäuren)** | | | | | | |
|---|---|---|---|---|---|---|
| Asparaginsäure (Asp) | 7188 | 6941 | 7287 | 10038 | 10948 | 10159 |
| Serin (Ser) | 2344 | 3104 | 3039 | 3161 | 2787 | 798 |
| Asparagin (Asn) | nn | nn | nn | nn | nn | nn |
| Glutaminsäure (Glu) | 12268 | 12614 | 12217 | 15289 | 16814 | 7777 |
| Glycin (Gly) | 1763 | 1274 | 1386 | 1800 | 2119 | 2186 |
| Alanin (Ala) | 4958 | 4383 | 4159 | 7320 | 7204 | 7185 |
| Cystein (Cys) | nicht bestimmt | | | nicht bestimmt | | |
| Tyrosin (Tyr) | 938 | 1316 | 1611 | 1522 | 1463 | 269 |
| Prolin (Pro) | 5506 | 5271 | 4993 | 8859 | 8653 | 9694 |
| Glutamin (Gln) | nicht bestimmt | | | nicht bestimmt | | |

| **weitere bestimmte Substanzen** | | | | | | |
|---|---|---|---|---|---|---|
| Taurin (Tau) | nicht auswertbar | nicht auswertbar | nicht auswert -bar | nicht auswertbar | nicht auswertbar | nicht auswertbar |
| Citrullin (Cit) | nn | nn | 130 | nn | nn | nn |
| Cystin (Cys2) | 1898 | 1175 | 1617 | 2515 | 3922 | 3235 |
| Cystathionin (Cystha) | nn | nn | nn | nn | nn | nn |
| 3-Methyl-Histidin (3Mehis) | nn | nn | nn | nn | nn | nn |
| 1-Methyl-Histidin (1Mehis) | nn | nn | nn | nn | nn | nn |
| Hydroxylysin (Hylys) | nn | nn | nn | nn | nn | nn |
| Ornithin (Orn) | nn | nn | 59 | nn | nn | 260 |
| Hydroxyprolin (Hypro) | nn | nn | nn | nn | nn | nn |
| | | | | | | |
| Summe | 70976 | 69191 | 70287 | 100247 | 103977 | 89723 |

| **Ergebnisse** | **EZ 44** | | | **EZ 45** | | |
|---|---|---|---|---|---|---|
| **freie Aminosäuren:** | **6h** | **21h** | **26h** | **6h** | **25,5 h** | **30,5 h** |
| | mg/kg Versuchsansatz | | | | | |

| **essentielle Aminosäuren (proteinogene Aminosäuren)** | | | | | | |
|---|---|---|---|---|---|---|
| Threonin (Thr) | 76 | 463 | 695 | 35 | 385 | 517 |
| Valin (Val) | 49 | 417 | 612 | 11 | 364 | 471 |
| Methionin (Met) | 170 | 398 | 532 | 100 | 286 | 317 |
| Isoleucin (Iso) | 33 | 246 | 481 | 34 | 220 | 301 |
| Leucin (Leu) | 576 | 1798 | 2731 | 237 | 1142 | 1592 |
| Phenylalanin (Phe) | 146 | 498 | 757 | 93 | 347 | 479 |
| Lysin (Lys) | 1141 | 2192 | 2961 | 691 | 1350 | 1831 |
| Tryptophan (Trp) | nicht bestimmt | | | nicht bestimmt | | |

| **semi-essentielle Aminosäuren (proteinogene Aminosäuren)** | | | | | | |
|---|---|---|---|---|---|---|
| Histidin (His) | 76 | 208 | 330 | 36 | 128 | 209 |
| Arginin (Arg) | 219 | 462 | 406 | 126 | 340 | 394 |

| **nicht-essentielle Aminosäuren (proteinogene Aminosäuren)** | | | | | | |
|---|---|---|---|---|---|---|
| Asparaginsäure (Asp) | 48 | 251 | 232 | 28 | 93 | 156 |
| Serin (Ser) | 61 | 257 | 384 | 39 | 219 | 278 |
| Asparagin (Asn) | nn | 220 | 356 | nn | 146 | 154 |
| Glutaminsäure (Glu) | 156 | 1070 | 2099 | 118 | 924 | 1382 |
| Glycin (Gly) | 10 | 94 | 152 | nn | 63 | 101 |
| Alanin (Ala) | 93 | 369 | 606 | 66 | 291 | 431 |
| Cystein (Cys) | nicht bestimmt | | | nicht bestimmt | | |
| Tyrosin (Tyr) | 80 | 382 | 684 | 27 | 250 | 412 |
| Prolin (Pro) | nn | 94 | 173 | nn | nn | 194 |
| Glutamin (Gln) | nicht bestimmt | | | nicht bestimmt | | |

| **weitere bestimmte Substanzen** | | | | | | |
|---|---|---|---|---|---|---|
| Taurin (Tau) | nicht auswertbar | nicht auswertbar | nicht auswertbar | nicht auswertbar | nicht auswertbar | nicht auswertbar |
| Citrullin (Cit) | nn | 58 | 105 | nn | nn | 58 |
| Cystin (Cys2) | 27 | 59 | 228 | nn | 23 | 55 |
| Cystathionin (Cystha) | 48 | 89 | 136 | 31 | 48 | nn |
| 3-Methyl-Histidin (3Mehis) | nn | nn | nn | nn | nn | nn |
| 1-Methyl-Histidin (1Mehis) | nn | nn | nn | nn | nn | nn |
| Hydroxylysin (Hylys) | nn | nn | nn | nn | nn | nn |
| Ornithin (Orn) | nn | 13 | 146 | nn | 29 | 54 |
| Hydroxyprolin (Hypro) | nn | nn | nn | nn | nn | 51 |
| | | | | | | |
| Summe | 3009 | 9638 | 14806 | 1672 | 6648 | 9437 |

| **Ergebnisse NPN-Extrakt** | **EZ 44** | | | **EZ 45** | | |
|---|---|---|---|---|---|---|
| **(Oliqopeptide und freie Aminosäuren):** | **6h** | **21h** | **26h** | **6h** | **25,5h** | **30,5h** |
| | mg/kg Versuchsansatz | | | | | |

| **essentielle Aminosäuren (proteinogene Aminosäuren)** | | | | | | |
|---|---|---|---|---|---|---|
| Threonin (Thr) | 5627 | 2394 | 6272 | 2782 | 4669 | 3677 |
| Valin (Val) | 3457 | 3624 | 3881 | 3236 | 3310 | 3361 |
| Methionin (Met) | 950 | 455 | 1300 | 444 | 714 | 510 |
| Isoleucin (Iso) | 3503 | 3849 | 4129 | 3443 | 3566 | 3598 |
| Leucin (Leu) | 7474 | 7984 | 8521 | 6684 | 6916 | 7196 |
| Phenylalanin (Phe) | 2091 | 1891 | 2503 | 1721 | 1955 | 1994 |
| Lysin (Lys) | 6759 | 7179 | 7806 | 5881 | 5975 | 6121 |
| Tryptophan (Trp) | nicht bestimmt | | | nicht bestimmt | | |

| **semi-essentielle Aminosäuren (proteinogene Aminosäuren)** | | | | | | |
|---|---|---|---|---|---|---|
| Histidin (His) | 1247 | 1419 | 1695 | 1115 | 1229 | 1327 |
| Arginin (Arg) | 1567 | 1349 | 1336 | 1264 | 1444 | 1332 |

| **nicht-essentielle Aminosäuren (proteinogene Aminosäuren)** | | | | | | |
|---|---|---|---|---|---|---|
| Asparaginsäure (Asp) | 6711 | 6333 | 8226 | 5793 | 6098 | 6405 |
| Serin (Ser) | 3763 | 1070 | 4374 | 1523 | 2960 | 2174 |
| Asparagin (Asn) | nn | nn | nn | nn | nn | nn |
| Glutaminsäure (Glu) | 13549 | 10107 | 14520 | 10546 | 11651 | 10480 |
| Glycin (Gly) | 1163 | 1324 | 1571 | 1010 | 1114 | 1208 |
| Alanin (Ala) | 4496 | 4273 | 4609 | 4061 | 3880 | 3918 |
| Cystein (Cys) | nicht bestimmt | | | nicht bestimmt | | |
| Tyrosin (Tyr) | 1379 | 545 | 1995 | 546 | 1322 | 1086 |
| Prolin (Pro) | 5490 | 5579 | 5819 | 5146 | 5035 | 4910 |
| Glutamin (Gln) | nicht bestimmt | | | nicht bestimmt | | |

| **weitere bestimmte Substanzen** | | | | | | |
|---|---|---|---|---|---|---|
| Taurin (Tau) | nicht auswertbar | nicht auswertbar | nicht auswertbar | nicht auswertbar | nicht auswertbar | nicht auswertbar |
| Citrullin (Cit) | nn | nn | nn | nn | nn | nn |
| Cystin (Cys2) | 742 | 1765 | 1276 | 1207 | 1059 | 1583 |
| Cystathionin (Cystha) | nn | nn | nn | nn | nn | nn |
| 3-Methyl-Histidin (3Mehis) | nn | nn | nn | nn | nn | nn |
| 1-Methyl-Histidin (1Mehis) | nn | nn | nn | nn | nn | nn |
| Hydroxylysin (Hylys) | nn | nn | nn | nn | nn | nn |
| Ornithin (Orn) | nn | nn | 139 | nn | nn | 64 |
| Hydroxyprolin (Hypro) | nn | nn | nn | nn | nn | nn |
| | | | | | | |
| Summe | 69968 | 61140 | 79972 | 56402 | 62897 | 60944 |

Die Proben wurden gezielt hinsichtlich ihres Geschmacks untersucht. Die Proben waren nicht bitter.

## Patentansprüche

1. Verfahren zur Herstellung einer Zusammensetzung aufweisend freie Aminosäuren, Oligopeptide und Polypeptide
**dadurch gekennzeichnet, dass**
das Verfahren zumindest den folgenden Schritt aufweist:
Zugabe von mindestens zwei verschiedenen Peptidasen, welche in aufgereinigter Form vorliegen, zu einer Zusammensetzung aufweisend mindestens ein Protein, wobei mindestens eine Exopeptidase und mindestens eine Endopeptidase zu der Zusammensetzung aufweisend mindestens ein Protein zugegeben werden, und wobei
(a) die mindestens zwei verschiedenen Peptidasen mikrobiellen Ursprungs sind;
(b) die Zusammensetzung aufweisend mindestens ein Protein eine Proteinkonzentration von 5 bis 30 Gewichtsprozent bezogen auf das Gesamtgewicht der Zusammensetzung aufweisend mindestens ein Protein aufweist;
(c) die Zusammensetzung aufweisend mindestens ein Protein bei Zugabe der mindestens zwei verschiedenen Peptidasen einen pH-Wert von 4 bis 6 hat und bei einer Temperatur von 40°C bis 60°C mit den mindestens zwei verschiedenen Peptidasen inkubiert wird; und
(d) die Gesamtmenge an zugegebener Peptidase jeweils von 2 bis 10 Gramm pro 100 Gramm des mindestens einen Proteins in der Zusammensetzung aufweisend mindestens ein Protein beträgt.

2. Verfahren gemäß Anspruch 1, **dadurch gekennzeichnet, dass** die mindestens zwei verschiedenen Peptidasen bakteriellen oder fungalen Ursprungs sind.

3. Verfahren gemäß mindestens einem der Ansprüche 1 oder 2, **dadurch gekennzeichnet, dass** die Zusammensetzung aufweisend mindestens ein Protein
(a) eine Proteinkonzentration von 10 bis 20 Gewichtsprozent bezogen auf das Gesamtgewicht der Zusammensetzung aufweisend mindestens ein Protein aufweist;
(b) für mindestens 1 h, vorzugsweise für eine Zeitspanne von 1 h bis 24 h, besonders bevorzugt für eine Zeitspanne von 4 h bis 12 h, am meisten bevorzugt für eine Zeitspanne von 6 h bis 10 h mit zumindest einer der mindestens zwei verschiedenen Peptidasen inkubiert wird;
(c) bei einer Temperatur von 45°C bis 60°C, besonders bevorzugt bei einer Temperatur von 45°C bis 55°C mit den mindestens zwei verschiedenen Peptidasen inkubiert wird;
(d) bei Zugabe der mindestens zwei verschiedenen Peptidasen einen pH-Wert von 4,5 bis 6 hat, wobei der pH-Wert gegebenenfalls durch die Zugabe mindestens einer organischen Säure, insbesondere ausgewählt aus Milchsäure, Zitronensäure und Äpfelsäure eingestellt wird; und/oder
(e) mindestens eine Eiweißquelle ausgewählt aus Molke, Molkeprotein-Konzentrat, Milchprotein-Konzentrat, Schlämpe aus der Lebensmittelproduktion wie zum Bespiel Destillerie-Schlämpe oder Brauerei-Schlämpe, Erbsen, Linsen, Bohnen, Soja und eine Kombination aus zwei oder mehreren hiervon aufweist.

4. Verfahren gemäß mindestens einem der vorhergehenden Ansprüche, **dadurch gekennzeichnet, dass** das Verfahren zusätzlich den folgenden Schritt aufweist: Zugabe von Calciumcarbonat und/oder Magnesiumcarbonat, wobei die Zugabe nach Abschluss der Inkubation der Zusammensetzung aufweisend mindestens ein Protein mit den mindestens zwei Peptidasen, erfolgt.

5. Verfahren gemäß mindestens einem der vorhergehenden Ansprüche, **dadurch gekennzeichnet, dass**
(a) die Gesamtmenge an zugegebener Peptidase jeweils von 500 bis 20.000, vorzugsweise von 1.000 bis 15.000, besonders bevorzugt von 2.000 bis 10.000 U pro 100 Gramm des mindestens einen Proteins in der Zusammensetzung aufweisend mindestens ein Protein beträgt;
(b) eine wiederholte Zugabe von mindestens einer der mindestens zwei Peptidasen zu der Zusammensetzung aufweisend mindestens ein Protein erfolgt;
(c) mindestens eine der mindestens zwei Peptidasen wiederholt in im Wesentlichen gleich großen Mengen und in im Wesentlichen gleich großen zeitlichen Abständen zu der Zusammensetzung aufweisend mindestens ein Protein zugegeben wird; und/oder
(d) die Zugabe der mindestens einen Endopeptidase vor der Zugabe der mindestens einen Exopeptidase erfolgt.

6. Verfahren gemäß mindestens einem der vorhergehenden Ansprüche, **dadurch gekennzeichnet, dass** die Zusammensetzung aufweisend mindestens ein Protein zuerst für 1 bis 10 h, vorzugsweise für 2 bis 6 h, besonders bevorzugt für etwa 5 h mit der mindestens einen Endopeptidase und anschließend für 3 bis 40 h, vorzugsweise für 20 bis 40 h, besonders bevorzugt für etwa 30 h mit der mindestens einen Exopeptidase inkubiert wird, wobei die Inkubation mit der Exopeptidase vorzugsweise in Gegenwart der mindestens einen Endopeptidase erfolgt.

7. Verfahren gemäß Anspruch 6, **dadurch gekennzeichnet, dass** die mindestens eine Endopeptidase und/oder die mindestens eine Exopeptidase wiederholt zugesetzt wird, vorzugsweise zweimal, dreimal, viermal, fünfmal oder sechsmal in im Wesentlichen gleich großen Mengen und in im Wesentlichen gleich großen zeitlichen Abständen zugesetzt wird.

8. Zusammensetzung aufweisend freie Aminosäuren, Oligopeptide und Polypeptide, erhältlich durch das Verfahren gemäß mindestens einem der vorhergehenden Ansprüche, **dadurch gekennzeichnet, dass**
die Zusammensetzung (a) von 0,5 Gewichtsprozent bis 25 Gewichtsprozent an freien Aminosäuren bezogen auf das Gesamtgewicht von freien Aminosäuren, Oligopeptiden, Polypeptiden und Proteinen ohne die zugesetzten Peptidasen aufweist und (b) von 25 bis 65 Gewichtsprozent an Oligopeptiden bezogen auf das Gesamtgewicht von freien Aminosäuren, Oligopeptiden, Polypeptiden und Proteinen ohne die zugesetzten Peptidasen aufweist.

9. Zusammensetzung gemäß Anspruch 8, **dadurch gekennzeichnet, dass**
(a) die Zusammensetzung von 2 Gewichtsprozent bis 20 Gewichtsprozent, besonders bevorzugt von 5 Gewichtsprozent bis 15 Gewichtsprozent an freien Aminosäuren bezogen auf das Gesamtgewicht von freien Aminosäuren, Oligopeptiden, Polypeptiden und Proteinen ohne die zugesetzten Peptidasen aufweist;
(b) die Zusammensetzung von 0,3 Gewichtsprozent bis 13 Gewichtsprozent, vorzugsweise von 1,2 Gewichtsprozent bis 12 Gewichtsprozent, besonders bevorzugt von 3 Gewichtsprozent bis 9 Gewichtsprozent an den folgenden freien Aminosäuren in Summe bezogen auf das Gesamtgewicht von freien Aminosäuren, Oligopeptiden, Polypeptiden und Proteinen ohne die zugesetzten Peptidasen aufweist: Isoleucin, Leucin, Lysin, Methionin, Phenylalanin, Threonin, Tryptophan und Valin;
(c) die Zusammensetzung einen pH-Wert von 4 bis 6, besonders bevorzugt von 4,5 bis 6 aufweist; und/oder
(d) das Gewichtsverhältnis von Magnesium zu Calcium in der Zusammensetzung von 1,25:1 bis 3:1, vorzugsweise von 1,5:1 bis 2,5:1, besonders bevorzugt etwa 2:1 beträgt.

10. Zusammensetzung gemäß Anspruch 8 oder 9, **dadurch gekennzeichnet, dass** 0 Gewichtsprozent bis 40 Gewichtsprozent, vorzugsweise 0 Gewichtsprozent bis 20 Gewichtsprozent, besonders bevorzugt 0 Gewichtsprozent bis 10 Gewichtsprozent des mindestens einen Proteins als ungespaltenes Protein vorliegt.

11. Verwendung der Zusammensetzung gemäß mindestens einem der Ansprüche 8 bis 10 zur Herstellung eines Nahrungsmittels, insbesondere zur Herstellung eines Fruchtsaftgetränks, eines Milchgetränks, eines Molkegetränks, eines Joghurts, eines Ayrans oder eines Kefirs.

12. Verwendung gemäß Anspruch 11, **dadurch gekennzeichnet, dass** ein Teil des Wassers eines Nahrungsmittels durch die Zusammensetzung gemäß mindestens einem der Ansprüche 8 bis 10 ersetzt wird.

13. Verwendung der Zusammensetzung gemäß mindestens einem der Ansprüche 8 bis 10 zur Herstellung eines Körperpflegeprodukts, insbesondere zur Herstellung einer Seife, eines Shampoos, einer Haarkur, einer Haarmaske, einer Haarpackung, eines Haarwassers, eines Conditioners, eines Badezusatzes, einer Body Lotion, eines Körpergels, einer Lotion zur Behandlung von Rosacea, einer Creme zur Akne Behandlung, einer Gesichtsmaske oder einer Gesichtscreme.

14. Verwendung gemäß Anspruch 13, **dadurch gekennzeichnet, dass** ein Teil des Wassers, vorzugsweise mindestens 80% des Wassers, eines Körperpflegeprodukts, vorzugsweise einer Seife, durch die Zusammensetzung gemäß mindestens einem der Ansprüche 8 bis 10 ersetzt wird.

15. Nahrungsmittel aufweisend die Zusammensetzung gemäß mindestens einem der Ansprüche 8 bis 10.

16. Nahrungsmittel gemäß Anspruch 15, **dadurch gekennzeichnet, dass** der Gesamtanteil an freien Aminosäuren, Oligopeptiden und Polypeptiden in dem Nahrungsmittel (a) von 2 Gewichtsprozent bis 25 Gewichtsprozent, vorzugsweise von 5 bis 10 Gewichtsprozent beträgt; und/oder (b) von 5 bis 50 Gramm, vorzugsweise etwa 10 Gramm beträgt.

17. Körperpflegeprodukt aufweisend die Zusammensetzung gemäß mindestens einem der Ansprüche 8 bis 10.

## Claims

1. A process for producing a composition comprising free amino acids, oligopeptides, and polypeptides
**characterized in that**
the process comprises at least the following step:
addition of at least two different peptidases, which are present in purified form, to a composition comprising at least one protein, wherein at least one exopeptidase and
at least one endopeptidase are added to the composition comprising at least one protein, and wherein
(a) the at least two different peptidases are of microbial origin;
(b) the composition comprising at least one protein has a protein concentration of 5 to 30 wt% based on the total weight of the composition comprising at least one protein;
(c) the composition comprising at least one protein has a pH of 4 to 6 when adding the at least two different peptidases and is incubated with the at least two different peptidases at a temperature of 40°C to 60°C; and
(d) the total amount of peptidase added is each from 2 to 10 grams per 100 grams of the at least one protein in the composition comprising at least one protein.

2. The process according to claim 1, **characterized in that** the at least two different peptidases are of bacterial or fungal origin.

3. The process according to any one of claims 1 or 2, **characterized in that** the composition comprising at least one protein
(a) has a protein concentration of 10 to 20 wt% based on the total weight of the composition comprising at least one protein;
(b) is incubated with at least one of the at least two different peptidases for at least 1 h, preferably for a period of 1 h to 24 h, more preferably for a period of 4 h to 12 h, most preferably for a period of 6 h to 10 h;
(c) is incubated with the at least two different peptidases at a temperature of 45°C to 60°C, more preferably at a temperature of 45°C to 55°C;
(d) has a pH of 4.5 to 6 when adding the at least two different peptidases, wherein the pH is optionally adjusted by addition of at least one organic acid preferably selected from lactic acid, citric acid, and malic acid; and/or
(e) comprises at least one protein source selected from whey, whey protein concentrate, milk protein concentrate, slurry from food production such as distillery slurry or brewery slurry, peas, lentils, beans, soybeans, and a combination of two or more thereof.

4. The process according to any one of the preceding claims, **characterized in that** the process also comprises the following step: addition of calcium carbonate and/or magnesium carbonate, wherein the addition takes place after the end of the incubation of the composition comprising at least one protein with the at least two peptidases.

5. The process according to any one of the preceding claims, **characterized in that**
(a) the total amount of peptidase added is each from 500 to 20,000, preferably from 1,000 to 15,000, more preferably from 2,000 to 10,000 U per 100 grams of the at least one protein in the composition comprising at least one protein;
(b) at least one of the at least two peptidases is repeatedly added to the composition comprising at least one protein;
(c) at least one of the at least two peptidases is repeatedly added in essentially equal amounts and in essentially equal time intervals to the composition comprising at least one protein; and/or
(d) the at least one endopeptidase is added prior to adding the at least one exopeptidase.

6. The process according to any one of the preceding claims, **characterized in that** the composition comprising at least one protein is first incubated with the at least one endopeptidase for 1 to 10 h, preferably for 2 to 6 h, more preferably for about 5 h, and then incubated with the at least one exopeptidase for 3 to 40 h, preferably for 20 to 40 h, more preferably for about 30 h, wherein the incubation with the exopeptidase is preferably in the presence of the at least one endopeptidase.

7. The process according to claim 6, **characterized in that** the at least one endopeptidase and/or the at least one exopeptidase is added repeatedly, preferably twice, three times, four times, five times, or six times, in essentially equal amounts and in essentially equal time intervals.

8. A composition comprising free amino acids, oligopeptides, and polypeptides, obtainable by the process according to any one of the preceding claims, **characterized in that**
the composition (a) comprises from 0.5 wt% to 25 wt% free amino acids, based on the total weight of free amino acids, oligopeptides, polypeptides, and proteins without the added peptidases and (b) comprises from 25 to 65 wt% oligopeptides, based on the total weight of free amino acids, oligopeptides, polypeptides, and proteins without the added peptidases.

9. The composition according to claim 8, **characterized in that**
(a) the composition comprises from 2 wt% to 20 wt%, more preferably from 5 wt% to 15 wt% free amino acids, based on the total weight of free amino acids, oligopeptides, polypeptides, and proteins without the added peptidases;
(b) the composition comprises from 0.3 wt% to 13 wt%, preferably from 1.2 wt% to 12 wt%, more preferably from 3 wt% to 9 wt% of the following free amino acids in total, based on the total weight of free amino acids, oligopeptides, polypeptides, and proteins without the added peptidases: isoleucine, leucine, lysine, methionine, phenylalanine, threonine, tryptophan, and valine;
(c) the composition has a pH of 4 to 6, more preferably of 4.5 to 6; and/or
(d) the weight ratio of magnesium to calcium in the composition is from 1.25:1 to 3:1, preferably from 1.5:1 to 2.5:1, more preferably about 2:1.

10. The composition according to any one of claims 8 or 9, **characterized in that** 0 wt% to 40 wt%, preferably 0 wt% to 20 wt%, more preferably 0 wt% to 10 wt% of the at least one protein is present as uncleaved protein.

11. Use of the composition according to any one of claims 8 to 10 for producing a food product, in particular for producing a fruit juice beverage, a milk beverage, a whey beverage, a yogurt, an ayran, or a kefir.

12. The use according to claim 11, **characterized in that** a portion of the water of a food product is replaced with the composition according to any one of claims 8 to 10.

13. Use of the composition according to any one of claims 8 to 10 for producing a personal care product, in particular for producing a soap, a shampoo, a hair treatment, a hair mask, a hair pack, a hair tonic, a conditioner, a bath essence, a body lotion, a body gel, a lotion for treating rosacea, a cream for treating acne, a facial mask or a facial cream.

14. The use according to claim 13, **characterized in that** a portion of the water, preferably at least 80% of the water, of a personal care product, preferably of a soap, is replaced with the composition according to any one of Claims 8 to 10.

15. A food product comprising the composition according to any one of claims 8 to 10.

16. The food product according to claim 15, **characterized in that** the total content of free amino acids, oligopeptides, and polypeptides in the food product is (a) from 2 wt% to 25 wt%, preferably from 5 to 10 wt%; and/or is (b) from 5 to 50 grams, preferably about 10 grams.

17. A personal care product comprising the composition according to any one of claims 8 to 10.

## Revendications

1. Procédé de fabrication d'une composition présentant des acides aminés libres, des oligopeptides et des polypeptides,
**caractérisé en ce que**
le procédé présente au moins l'étape suivante :
l'addition d'au moins deux peptidases différentes, qui sont présentes sous forme purifiée, à une composition présentant au moins une protéine, dans lequel au moins une exopeptidase et au moins une endopeptidase sont ajoutées à la composition présentant au moins une protéine, et dans lequel
(a) les au moins deux peptidases différentes sont d'origine microbienne ;
(b) la composition présentant au moins une protéine présente une concentration en protéines de 5 à 30 % en poids par rapport au poids total de la composition présentant au moins une protéine ;
(c) la composition présentant au moins une protéine a un pH de 4 à 6 lors de l'ajout des au moins deux peptidases différentes et est incubée avec les au moins deux peptidases différentes à une température de 40 °C à 60 °C ; et
(d) la quantité totale de peptidase ajoutée va respectivement de 2 à 10 grammes par 100 grammes de l'au moins une protéine dans la composition présentant au moins une protéine.

2. Procédé selon la revendication 1, **caractérisé en ce que** les au moins deux peptidases différentes sont d'origine bactérienne ou fongique.

3. Procédé selon au moins l'une quelconque des revendications 1 ou 2, **caractérisé en ce que** la composition présentant au moins une protéine
(a) présente une concentration en protéines de 10 à 20 % en poids par rapport au poids total de la composition présentant au moins une protéine ;
(b) est incubée avec au moins l'une des au moins deux peptidases différentes pendant au moins 1 h, de préférence pendant une durée de 1 h à 24 h, de manière particulièrement préférée pendant une durée de 4 h à 12 h, idéalement pendant une durée de 6 h à 10 h ;
(c) est incubée avec les au moins deux peptidases différentes à une température de 45 °C à 60 °C, de manière particulièrement préférée à une température de 45 °C et 55 °C ;
(d) présente un pH de 4,5 à 6 lors de l'addition des au moins deux peptidases différentes, dans lequel le pH est ajusté éventuellement par l'addition d'au moins un acide organique, en particulier choisi parmi l'acide lactique, l'acide citrique et l'acide malique ; et/ou
(e) présente au moins une source de protéines choisie parmi le lactosérum, le concentré de protéines de lactosérum, le concentré de protéines de lait, les drêches issues de la production alimentaire telles que les drêches de distillerie ou de brasserie, les pois, les lentilles, les haricots, le soja et une combinaison de deux ou plusieurs d'entre eux.

4. Procédé selon au moins l'une quelconque des revendications précédentes, **caractérisé en ce que** le procédé présente en supplément l'étape suivante : l'addition de carbonate de calcium et/ou de carbonate de magnésium, dans lequel l'addition est effectuée à la fin de l'incubation de la composition présentant au moins une protéine avec les au moins deux peptidases.

5. Procédé selon au moins l'une quelconque des revendications précédentes, **caractérisé en ce que**
(a) la quantité totale de peptidase ajoutée est respectivement de 500 à 20 000, de préférence de 1 000 à 15 000, de manière particulièrement préférée de 2 000 à 10 000 U pour 100 grammes de l'au moins une protéine dans la composition présentant au moins une protéine ;
(b) une addition répétée d'au moins une des au moins deux peptidases à la composition présentant au moins une protéine est effectuée ;
(c) au moins une des au moins deux peptidases est ajoutée à plusieurs reprises en des quantités sensiblement identiques et à des intervalles sensiblement identiques à la composition présentant au moins une protéine ; et/ou
(d) l'addition de l'au moins une endopeptidase est effectuée avant l'addition de l'au moins une exopeptidase.

6. Procédé selon au moins l'une quelconque des revendications précédentes, **caractérisé en ce que** la composition présentant au moins une protéine est incubée d'abord avec l'au moins une endopeptidase pendant 1 à 10 h, de préférence pendant 2 à 6 h, de manière particulièrement préférée pendant environ 5 h, puis avec l'au moins une exopeptidase pendant 3 à 40 h, de préférence pendant 20 à 40 h, de manière particulièrement préférée pendant environ 30 h, dans lequel l'incubation avec l'exopeptidase est effectuée de préférence en présence de l'au moins une endopeptidase.

7. Procédé selon la revendication 6, **caractérisé en ce que** l'au moins une endopeptidase et/ou l'au moins une exopeptidase est ajoutée de manière répétée, de préférence deux, trois, quatre, cinq ou six fois en des quantités sensiblement identiques et à des intervalles sensiblement identiques.

8. Composition présentant des acides aminés libres, des oligopeptides et des polypeptides, pouvant être obtenue par le procédé selon au moins l'une quelconque des revendications précédentes, **caractérisée en ce que** la composition présente (a) de 0,5 % en poids à 25 % en poids d'acides aminés libres par rapport au poids total d'acides aminés libres, d'oligopeptides, de polypeptides et de protéines sans les peptidases ajoutées et (b) de 25 à 65 % en poids d'oligopeptides par rapport au poids total d'acides aminés libres, d'oligopeptides, de polypeptides et de protéines sans les peptidases ajoutées.

9. Composition selon la revendication 8, **caractérisée en ce que**
(a) la composition présente de 2 % en poids à 20 % en poids, de manière particulièrement préférée de 5 % en poids à 15 % en poids d'acides aminés libres par rapport au poids total d'acides aminés libres, d'oligopeptides, de polypeptides et de protéines sans les peptidases ajoutées ;
(b) la composition présente de 0,3 % en poids à 13 % en poids, de préférence de 1,2 % en poids à 12 % en poids, de manière particulièrement préférée de 3 % en poids à 9 % en poids des acides aminés libres qui suivent au total par rapport au poids total d'acides aminés libres, d'oligopeptides, de polypeptides et de protéines sans les peptidases ajoutées : isoleucine, leucine, lysine, méthionine, phénylalanine, thréonine, tryptophane et valine ;
(c) la composition présente un pH de 4 à 6, de manière particulièrement préférée de 4,5 à 6 ; et/ou
(d) le rapport de poids entre le magnésium et le calcium dans la composition va de 1,25:1 à 3:1, de préférence de 1,5:1 à 2,5:1, est de manière particulièrement préférée d'environ 2:1.

10. Composition selon la revendication 8 ou 9, **caractérisée en ce que** 0 % en poids à 40 % en poids, de préférence 0 % en poids à 20 % en poids, de manière particulièrement préférée 0 % en poids à 10 % en poids de l'au moins une protéine sont présents en tant que protéine non clivée.

11. Utilisation de la composition selon au moins l'une quelconque des revendications 8 à 10 pour la fabrication d'une denrée alimentaire, notamment pour la fabrication d'une boisson à base de jus de fruits, d'une boisson lactée, d'une boisson à base de lactosérum, d'un yaourt, d'un ayran ou d'un kéfir.

12. Utilisation selon la revendication 11, **caractérisée en ce qu'**une partie de l'eau d'une denrée alimentaire est remplacée par la composition selon au moins l'une quelconque des revendications 8 à 10.

13. Utilisation de la composition selon au moins l'une quelconque des revendications 8 à 10 pour la fabrication d'un produit de soin corporel, en particulier pour la fabrication d'un savon, d'un shampooing, d'un produit de soin capillaire, d'un masque capillaire, d'un bonnet pour soin capillaire, d'une lotion capillaire, d'un aprèsshampooing, d'un additif de bain, d'une lotion pour le corps, d'un gel pour le corps, d'une lotion pour le traitement de la rosée, d'une crème pour le traitement de l'acné, d'un masque facial ou d'une crème pour le visage.

14. Utilisation selon la revendication 13, **caractérisée en ce qu'**une partie de l'eau, de préférence au moins 80 % de l'eau, d'un produit de soin corporel, de préférence d'un savon, est remplacée par la composition selon au moins l'une quelconque des revendications 8 à 10.

15. Denrée alimentaire présentant la composition selon au moins l'une quelconque des revendications 8 à 10.

16. Denrée alimentaire selon la revendication 15, **caractérisée en ce que** la teneur totale en acides aminés libres, oligopeptides et polypeptides dans la denrée alimentaire est (a) de 2 % en poids à 25 % en poids, de préférence de 5 à 10 % en poids ; et/ou (b) de 5 à 50 g, de préférence d'environ 10 g.

17. Produit de soin corporel présentant la composition selon au moins l'une quelconque des revendications 8 à 10.
